# EUROPÄISCHE PATENTANMELDUNG

(11) **EP 3 120 796 A1**
(43) Veröffentlichungstag der Anmeldung: **25.01.2017**
(21) Anmeldenummer: 16179586.9
(22) Anmeldetag: 14.07.2016
(51) Int. Cl.: A61B 34/10, A61C 13/00, A61F 2/30, A61B 17/00, A61B 17/80

(54) **VERFAHREN UND SYSTEM ZUR HERSTELLUNG EINES IMPLANTATS**

(30) Priorität: 17.07.2015 EP 15177415
(71) Anmelder: Mimedis AG, 4057 Basel (CH)
(72) Erfinder: Schumacher, Ralf, 4411 Seltisberg (CH); Coigny, Florian, 4132 Muttenz (CH); Schkommodau, Erik, 4410 Liestal (CH)
(74) Vertreter: Herrmann, Johanna

(57) **Zusammenfassung**

1. Ein Verfahren zur Bereitstellung eines patientenspezifischen Implantats wird in mehreren Schritten mit einem System in weitgehend automatisierter Form durchgeführt. Eine Verletzung eines Körperteils eines Patienten wird mittels eines bildgebenden Verfahrens gemessen, ein Messdatensatz des verletzten Körperteils erzeugt, der einem mehrdimensionalen Abbild der Verletzung des Körperteils entspricht, die mit dem bildgebenden Verfahren ermittelt worden ist. Aus dem Messdatensatz wird ein mehrdimensionales Modell des verletzten Körperteils erzeugt, wobei mit Hilfe des mehrdimensionalen Modells des verletzten Körperteils ein Modell des gesunden Körperteils erzeugt werden kann, welches insbesondere die Kräfte enthält, welche durch eine Dauerbelastung auf das Körperteil wirken. Ein mehrdimensionales Modell des Implantats wird erstellt, welches an das mehrdimensionale Modell des Körperteils angepasst wird, sodass ein modifiziertes mehrdimensionales Modell des Implantats erhalten wird. Das modifizierte mehrdimensionale Modell des Implantats wird aus mindestens einem Modulelement generiert. Die Festigkeit des Modulelements wird so ausgewählt, dass sie mindestens der Festigkeit bei einer maximalen Belastung des Körperteils entspricht, sodass die Belastung, der das Implantat im Gebrauchszustand über seine gesamte Lebensdauer maximal ausgesetzt ist, kleiner als die Dauerfestigkeit des Modulelements ist.

## Beschreibung

Die Erfindung betrifft ein Verfahren sowie ein System und ein Computerprogrammprodukt zur Herstellung eines Implantats, insbesondere zur Modellierung eines derartigen Implantats, welches beispielsweise mittels eines subtraktiven oder additiven Herstellungsverfahrens hergestellt werden kann. Bei dem Implantat handelt es sich um ein patientenspezifisches Implantat. Das Verfahren, das System sowie das Computerprogrammprodukt umfassen neben der eigentlichen Herstellung auch die vorgelagerten Schritte des Erstellens eines digitalen Modells des Implantats aufgrund von Patientendaten.

Aus dem Stand der Technik ist es bekannt, patientenspezifische Implantate mittels subtraktiven Verfahren, beispielsweise Bohren, Drehen, Fräsen herzustellen. Aus dem Stand der Technik ist aus der US 6772026 B2 ein Verfahren zum Erstellen eines individualisierten, anatomisch geformten Implantats für einen Patienten bekannt. Zur Herstellung eines derartigen individualisierten Implantats werden Daten aus Röntgenaufnahmen, MRI oder CT, der Struktur eines anatomischen Körperteils, nachfolgend als Strukturdaten bezeichnet, erstellt. Mittels der Daten aus den Röntgenaufnahmen wird ein mehrdimensionales digitales Modell zumindest eines Teils des anatomischen Körperteils, erstellt. Dieses mehrdimensionale digitale Modell wird modifiziert, um zumindest ein erstes mehrdimensionales Nachfolgemodell zu erstellen. Die Modifikation des mehrdimensionalen, digitalen Modells umfasst die Anpassung einer Materialdichteabstufung zumindest eines Teils des mehrdimensionalen digitalen Modells auf Basis der Materialdichteabstufung von ähnlichen anatomischen Körperteilen. Das mehrdimensionale Nachfolgemodell enthält zumindest eine physikalische Eigenschaft des anatomischen Körperteils, welcher nicht in den Röntgendaten enthalten ist. Das Verfahren gemäss US 6772026 B2 sieht vor, dass die Strukturdaten von einem Client-Computer über ein Computernetzwerk an einen Hostcomputer übertragen werden. Der Hostcomputer umfasst eine Workstation, also einen CAD-Arbeitsplatz. Die Strukturdaten werden am CAD-Arbeitsplatz von einem CAD-Designer in die CAD-Software eingelesen und können mit der CAD Software bearbeitet werden. Diese Vorgehensweise hat den Nachteil, dass die Bearbeitungszeit zur Erstellung eines mehrdimensionalen digitalen Modells aus den Strukturdaten je nach Verfügbarkeit des CAD-Technikers Schwankungen unterliegen kann. Daher kommt es zu Wartezeiten für den Patienten, da der behandelnde Arzt die Erstellung des mehrdimensionalen digitalen Modells abwarten muss. Nach der Erstellung des mehrdimensionalen digitalen Modells erfolgt die Modifikation desselben ebenfalls am CAD-Arbeitsplatz. Hierzu kann wiederum eine Abstimmung zwischen dem behandelnden Arzt und dem CAD-Techniker erforderlich sein, sodass auch während dieses Arbeitsablaufs weitere zeitliche Verzögerungen auftreten können. Zudem müssen die Strukturdaten in maschinenlesbare Daten konvertiert werden, bevor sie von der CAD Software eingelesen werden können. Hierzu kann es erforderlich sein, grosse Datenvolumina über Netzwerke zu übertragen.

Das Dokument DE20 2005 005 085 U1 beschreibt ein Verfahren, gemäss welchem ein Implantat aufgrund von Strukturdaten, die durch Messungen eines Defekts in einem Knochen oder Knorpel eines Patienten generiert worden sind, bearbeitet wird. Des Weiteren kann mittels der Strukturdaten das Volumen der zu ersetzenden Knorpelzellen ermittelt werden, sodass genau die Menge an Knorpelzellen gezüchtet werden kann, die für das Einwachsen des Implantat mit dem körpereigenen Zellgewebe benötigt wird. Allerdings wird mit diesem Verfahren kein Implantat aus einem Modell erzeugt. Aus den genannten Strukturdaten wird kein Modell generiert wird, weil nur Daten des Defektvolumens, z.B. dessen Fläche oder Höhe erfasst werden, diese Strukturdaten aber nicht Eingang in die Erstellung eines mehrdimensionalen digitalen Modells des Defekts finden. Daher erscheint das Verfahren nicht geeignet, ein mehrdimensionales digitales Modell des Defekts zu erstellen, welches für ein additives Herstellungsverfahren geeignet wäre.

Es wurde auch in der US8166627 B2 vorgeschlagen, ein dreidimensionales Modell eines Kiefergelenks durch Simulation anhand der Daten eines gesunden, gleichartigen Kiefergelenks zu erzeugen. Insbesondere kann diese Simulation eine Spiegelung des gesunden Kiefergelenkteils umfassen. Ein Ersatzgelenk kann unter Verwendung des dreidimensionalen Modells stereolithographisch erzeugt werden.

Es ist aus der WO2013/091085 A1 bekannt, ein aus Zellen aufgebautes Knochenimplantat herzustellen, welches eine nichthomogene Verteilung der Materialeigenschaften aufweist, wobei in einem Schritt ein Finite-Element-Modell (FEM) des Implantats erstellt wird, welches aus einer Mehrzahl von Einheitszellen aufgebaut ist, die eine Gittermikrostruktur bilden. Für jede Einheitszelle wird ein homogenisierter Steifigkeitstensor berechnet. Danach wird für jede Einheitszelle ein homogenes Medium definiert, das einen äquivalenten homogenisierten Steifigkeitstensor aufweist. Mittels einer Finite-Elemente-Analyse werden die mittleren makroskopischen Spannungen (Druck-, Zug-, Schubspannungen) unter Verwendung der homogenisierten Steifigkeitstensoren ermittelt, die auf das Implantat einwirken. Für jede Einheitszelle wird ein mikroskopisches Spannungsfeld erzeugt, welches durch ein Spannungs-Erholungsverfahren ermittelt wird, welches auf die makroskopischen Spannungen angewendet wird. Wenn das mikroskopische Spannungsfeld unterhalb eines vordefinierten Versagensfalls liegt, kann eine komplexe (i.e. auf der gleichzeitigen Optimierung unterschiedlicher Parameter) beruhende Optimierung zur Minimierung des Verlusts an Knochensubstanz und zur Vermeidung eines Versagens im Übergangsbereich zwischen Knochen und Implantat durchgeführt werden, wobei die mittlere Porosität, die mittlere Porengrösse sowie die Wandstärke für jede Einheitszelle optimiert werden. Aus der Summe der derart optimierten Spannungsfelder in den Einheitszellen wird ein Modell eines aus Zellen aufgebauten Implantats erstellt, welches das optimierte Spannungsfeld jeder Einheitszelle enthält. Die Zellen weisen eine Porosität von mindestens 40% auf. Die mittlere Porengrösse soll vorzugsweise zwischen 50 µm und 800 µm und die Dicke der Zellwände in einem Bereich zwischen 70 µm und 100 µm liegen.

Aus der FR 2 979 817 A1 ist die Herstellung eines patientenspezifischen Implantats bekannt, wobei ein mehrdimensionales Modell des Implantats an das mehrdimensionale Modell des verletzten oder gesunden Körperteils angepasst wird, sodass ein modifiziertes mehrdimensionales Modell des Implantats erhalten wird, wobei das modifizierte mehrdimensionale Modell des Implantats in eine Druckdatei für ein additives Herstellungsverfahren umgewandelt wird, wobei aus der Druckdatei Anweisungen zur schichtweisen Herstellung des Implantats durch ein additives Herstellungsverfahren in einer additiven Herstellungsvorrichtung erstellt werden, sodass das Implantat in der additiven Herstellungsvorrichtung hergestellt wird, wobei durch die Laserleistung sowie das verwendete Pulver ein Implantat mit geringer Oberflächenrauigkeit hergestellt wird. Als Pulvermaterialien werden Kobalt, Chrom, Titan oder Aluminium genannt. In den Anfängen der additiven Herstellungsverfahren, wie beispielsweise in der WO95/07509 A1 offenbart, lagen die Schichtdicken bei 0.25 mm pro Schicht, sodass ein patientenspezifisches Implantat geringer Oberflächenrauigkeit gar nicht erhältlich war. Erst durch die Weiterentwicklung der additiven Herstellungsverfahren wurden gemäss FR 2 979 817 A1 geringere Schichtdicken im Bereich von 0.025 bis 0.075 mm ermöglicht, wodurch die Oberflächenqualität des Implantats entsprechend verbessert werden konnte.

Die Nutzung eines Computersystems mit einer Client-Server Struktur ist gemäss US20020059049 A1 erforderlich, da ein Datenaustausch zwischen dem Ort der Diagnose und Behandlung, dem Ort der Modellierung des Implantats sowie dem Ort der Herstellung des Implantats erforderlich waren, wobei diese Orte sich voneinander unterscheiden. Die Kompetenz der Modellierung des Implantats und die Kompetenz zur Herstellung desselben befanden sich bisher nicht am Ort der Diagnose, d.h. in einer Arztpraxis oder einem Spital. Obwohl der Einsatz von Computersystemen und Netzwerken, wie dem Internet, den Austausch der Daten ermöglichen, ist dennoch ein Transfer an Informationen zwischen dem Arzt und dem Modellentwickler sowie dem Implantathersteller erforderlich. An diesem Austausch sind mindestens zwei Personen beteiligt, was dazu führt, dass zumindest diese beiden Personen sich terminlich abstimmen müssen, um sämtliche der in FR 2 979 817 A1 offenbarten Verfahrensschritte auszuführen. Hierdurch kommt es zu Wartezeiten, die für einen Patienten einen längeren Spitalaufenthalt und somit erhöhte Kosten zur Folge haben können.

Aus der WO2011056995 A2 ist es bekannt, patientenspezifische Knieimplantate herzustellen, die auf die Anatomie des Patienten, die Kinematik, das Gewebeumfeld (tissue impingement) abgestimmt sind. Hierbei wird allerdings nur die Passform des Implantats, insbesondere dessen Krümmung, also ein Mass für eine Linie auf dessen Oberfläche berücksichtigt. Das heisst, aus diesem Dokument ist nicht entnehmbar, welche mechanischen Eigenschaften ein derartiges Implantat aufweisen muss, um einer mehrjährigen Belastung im Körper standzuhalten.

US2013166256 A beschreibt eine Vorrichtung, mittels welcher ein dreidimensionales Modell eines Hüftknochens für die Herstellung einer chirurgischen Führung oder eines chirurgischen Werkzeugs für eine Operation an diesem Hüftknochen erstellt werden kann. Das dreidimensionale Modell des Hüftknochens wird mittels einer Genauigkeitskarte (accuracy map) überprüft, ob es innerhalb eines bestimmten Genauigkeitsbereichs liegt und es wird mittels der Vorrichtung ermittelt, ob die durch Simulation generierte chirurgische Führung innerhalb eines Stabilitätsbereichs liegt, wobei bei Abweichungen vom Stabilitätsbereichs die chirurgische Führung derart angepasst werden kann, dass sie im Stabilitätsbereich zu liegen kommt, wobei das Werkzeug bzw. das chirurgische Führungselement in Bezug auf das dreidimensionale Modell des Hüftknochens innerhalb eines vorbestimmten Toleranzbereichs liegen muss. Der Stabilitätsbereich sollte innerhalb eines Stabilitätswertebereichs (stability score) liegen. Die Genauigkeitskarte und/oder der Stabilitätswertebereich kann aus einem Speicher ermittelt werden, sodass verschiedene Designs der Vorrichtung, also des Werkzeugs oder des Führungselements für einen chirurgischen Eingriff, in Bezug auf die Stabilität der Vorrichtung analysiert werden, wobei unter Stabilität die Stabilität der Vorrichtung auf einer Kontaktoberfläche eines physikalischen Objekts, womit die Oberfläche eines Knochens gemeint sein könnte, zu verstehen sei. Dies ist nach unserer Auffassung so zu interpretieren, dass eine patientenspezifische Oberfläche des Werkzeugs oder Führungselements für den chirurgischen Eingriff erzeugt wird, indem nach einer Registrierung von zwei dreidimensionalen Datensätzen die Übereinstimmung eines jeden Punktes des ersten dreidimensionalen Modells mit dem jeweils korrespondierenden Punkt des zweiten dreidimensionalen Modells zu finden. Unter Registrierung der zwei dreidimensionalen Datensätze wird das Ausrichten der zwei dreidimensionalen Modelle verstanden, welches dem Ziel dient, die beiden dreidimensionalen Modelle möglichst flächendeckend zueinander auszurichten. Eine derartige Registrierung sowie die Verwendung einer Genauigkeitskarte werden auch von herkömmlichen dreidimensionalen Scannern verwendet. Da in diesem Dokument nur auf die Genauigkeit der Oberfläche des Werkzeugs in Bezug auf den Knochen abgestellt wird, ist die Vorrichtung gemäss US2013166256A1 nicht dazu geeignet, ein Modell zu erzeugen, dass die mechanischen Eigenschaften, z.B. Zugspannungen, Druckspannungen, Torsionskräfte und dergleichen im Inneren eines Implantats abbildet. Da sich die US2013166256A1 somit mit der Modellierung eines chirurgischen Werkzeugs oder eines chirurgischen, orthopädischen oder prothetischen Führungselements befasst, welches patientenspezifisch ausgebildet ist und demzufolge voraussichtlich nur ein einziges Mal für eine einzige OP zum Einsatz kommt, sind die mechanischen Eigenschaften im Volumenkörper eines derartigen Werkzeugs für die vorliegende Verwendung nicht vergleichbar, da ein Werkzeug gemäss US2013166256 A1 keiner Langzeitbelastung im Körper ausgesetzt ist.

Die Formstabilität des Werkzeugs oder Führungselements wird gemäss US2013166256 A1 nur bezogen auf die Kontaktfläche mit einem physischen Objekt betrachtet, wobei gemäss der Lehre der US2013166256 A1 die Formstabilität als der Widerstand definiert wird, der bei einem Kontakt zwischen dem Werkzeug oder Führungselement mit dem physischen Objekt bei Einwirkung einer von aussen wirkenden Kraft entsteht.

Daher ist es aufgrund der Verwendung des Werkzeugs oder Führungselements der US2013166256 A1 nicht möglich, Rückschlüsse auf die im Inneren des Werkzeugs oder Führungselements wirkenden Kräfte zu ziehen. Aufgrund des kurzzeitigen Einsatzes, oftmals des einmaligen Gebrauchs, besteht grundsätzlich keine Notwendigkeit, die im Inneren des Werkzeugs oder Führungselements wirkenden Kräfte zu ermitteln.

Für ein Implantat, welches eine tragende Funktion im Körper über einen möglichst langen Zeitraum ausüben können soll, ist aber die Kenntnis der im Implantat wirkenden Kräfte erforderlich, da hieraus beispielsweise das Ermüdungsbruchverhalten ermittelt werden kann. Dieses Ermüdungsbruchverhalten kann Hinweise über die natürliche biomechanische Belastung liefern, die während der gesamten Lebenserwartung eines Implantats im implantierten Zustand auftritt.

Aufgabe der Erfindung ist es, ein Verfahren sowie ein System zur Bereitstellung eines beliebig geformten Implantats vorzuschlagen, mittels welchem die Wartezeiten erheblich verringert werden können, weil das Implantat von einem Projektmitarbeiter nach Erhalt eines Messdatensatzes in einem zusammenhängenden Arbeitsablauf entworfen und hergestellt werden kann. Insbesondere wird das Verfahren und System derart vereinfacht, dass der Arzt unmittelbar nach Stellen der Diagnose mit den bei der Diagnose generierten Informationen bzw. Daten in die Lage versetzt wird, das Implantat zu entwerfen und dessen Herstellung in Auftrag zu geben.

Eine weitere Aufgabe der Erfindung ist es, eine Vorrichtung sowie ein Verfahren zur Herstellung eines beliebig geformten Implantats vorzuschlagen, bei dessen Auslegung die im Implantat während dessen bestimmungsgemässer Verwendung wirkenden Kräfte, Spannungen sowie Belastungszustände berücksichtigt werden können.

Zur Lösung dieser Aufgabe wird ein Baukastensystem vorgeschlagen, aus welchem der Projektmitarbeiter das für die gewünschte Verwendung optimale Implantat auswählen kann. Dieses Implantat kann vom Projektmitarbeiter in einem zusammenhängenden Arbeitsablauf entworfen und unter Berücksichtigung der Langzeitbelastung im Körper hergestellt werden.

Die Aufgabe der Erfindung wird durch ein Verfahren gemäss Anspruch 1 gelöst. Vorteilhafte Ausführungsbeispiele des Verfahrens sind Gegenstand der abhängigen Ansprüche 2 bis 7. Ein System zur Herstellung eines Implantats ist Gegenstand der Ansprüche 8 bis 15.

Wenn der Begriff "beispielsweise" in der nachfolgenden Beschreibung verwendet wird, bezieht sich dieser Begriff auf Ausführungsbeispiele und/oder Ausführungsformen, was nicht notwendigerweise als eine bevorzugtere Anwendung der Lehre der Erfindung zu verstehen ist. In ähnlicher Weise sind die Begriffe "vorzugsweise", "bevorzugt" zu verstehen, indem sie sich auf ein Beispiel aus einer Menge von Ausführungsbeispielen und/oder Ausführungsformen beziehen, was nicht notwendigerweise als eine bevorzugte Anwendung der Lehre der Erfindung zu verstehen ist. Dementsprechend können sich die Begriffe "beispielsweise", "vorzugsweise" oder "bevorzugt" auf eine Mehrzahl von Ausführungsbeispielen und/oder Ausführungsformen beziehen.

Die nachfolgende detaillierte Beschreibung enthält verschiedene Ausführungsbeispiele für das erfindungsgemässe Verfahren sowie das zugehörige System und Computerprodukt. Das Verfahren kann mittels unterschiedlicher Software ausgeführt werden, das System kann mittels unterschiedlicher Software betrieben werden sowie das Computerprogrammprodukt kann unterschiedliche Software enthalten, das sodass die Beschreibung einer bestimmten Software nur als beispielhaft anzusehen ist. In der Beschreibung und den Ansprüchen werden die Begriffe "enthalten", "umfassen", "aufweisen" als "enthalten, aber nicht beschränkt auf.." interpretiert.

Die Aufgabe der Erfindung wird durch ein Verfahren zur Herstellung eines patientenspezifischen Implantats gelöst, wobei in einem ersten Schritt eine Verletzung eines Körperteils eines Patienten mittels eines bildgebenden Verfahrens gemessen wird. In einem zweiten Schritt wird ein Messdatensatz erzeugt, der einem mehrdimensionalen Abbild der Verletzung des Körperteils entspricht, der mit dem bildgebenden Verfahren ermittelt worden ist. In einem dritten Schritt wird aus dem Messdatensatz ein mehrdimensionales Modell der Verletzung des Körperteils erzeugt. In einem vierten Schritt wird mit Hilfe des mehrdimensionalen Modells der Verletzung des Körperteils ein Modell des gesunden Körperteils erzeugt, wobei eine Spiegelung des Abbilds des gesunden Körperteils in das Abbild des verletzten Körperteils erfolgen kann. In einem fünften Schritt wird ein mehrdimensionales Modell des Implantats erstellt. In einem sechsten Schritt wird das mehrdimensionale Modell des Implantats an das mehrdimensionale Modell des verletzten oder gesunden Körperteils angepasst, sodass ein modifiziertes mehrdimensionales Modell des Implantats erhalten wird. Gegebenenfalls wird das mehrdimensionale Modell des Implantats in einem oder mehreren Iterationsschritten aufgrund einer Berechnung der Belastung des Körperteils derart verändert, dass das mehrdimensionale Modell des Implantats den Belastungen des Körperteils bestmöglich standhält und/oder die Befestigung des Implantats am Körperteil eine minimale Abnützung des Körperteils zur Folge hat. In einem siebenten Schritt wird das modifizierte mehrdimensionale Modell des Implantats in eine Druckdatei für additives Herstellungsverfahren umgewandelt. Insbesondere kann die Druckdatei für den Betrieb in einer additiven Herstellungsvorrichtung verwendet werden. Die Druckdatei kann Anweisungen zur schichtweisen Erzeugung des Implantats enthalten. In einem achten Schritt werden aus der Druckdatei Anweisungen zur schichtweisen Herstellung des Implantats durch ein additives Herstellungsverfahren in einer additiven Herstellungsvorrichtung erstellt. In einem neunten Schritt wird das Implantat in der additiven Herstellungsvorrichtung hergestellt. Insbesondere kann das Implantat schichtweise gemäss den in der Druckdatei für das additive Herstellungsverfahren bereitgestellten Anweisungen zur Herstellung des Implantats hergestellt werden. Im fünften Schritt wird mit einem Implantatauswahlmodul ein mehrdimensionales Modell eines Implantats ausgewählt oder im sechsten Schritt ein mehrdimensionales Modell eines Implantats mit einem Implantatgenerierungsmodul erzeugt, wobei das mehrdimensionale Modell des Implantats an das mehrdimensionale Modell des verletzten oder gesunden Körperteils angepasst wird, sodass das modifizierte mehrdimensionale Modell des Implantats erhalten wird. Sämtliche Schritte können hierbei an einem Arbeitsplatz von einer Person ausgeführt werden. Daher ist es möglich, dass der Arzt bereits im Anschluss an die Diagnose selbst aus dem Messdatensatz das mehrdimensionale Modell des Implantats erstellt sowie das Modell modifiziert, sodass das modifizierte mehrdimensionale Modell des Implantats erhalten wird. Insbesondere kann das mehrdimensionale Modell des Implantats mit einer für die Modellierung zum Einsatz kommenden Software für die relevante mechanische Belastung im Einsatz ausgelegt werden. Die Software enthält integrierte Designrichtlinien um Stabilität und Herstellbarkeit des Implantats zu garantieren, welches das Ergebnis der Modellierung darstellt. Parameterkombinationen, welche zu einem instabilen oder nicht herstellbaren Implantat führen würden, werden von der Software verhindert. Daher muss sich der
Nach einem Ausführungsbeispiel kann zumindest eines der mehrdimensionalen Modelle des verletzten Körperteils, des gesunden Körperteils oder des Implantats in der Perspektive des Betrachters in einer zweidimensionalen Bildebene manipuliert werden. Insbesondere kann die Manipulation ein Durchtrennen oder Schneiden, ein Verschieben oder ein Rotieren zumindest eines der mehrdimensionalen Modelle des verletzten Körperteils, des gesunden Körperteils oder des Implantats umfassen.

Im zweiten Schritt kann das Messergebnis nach Erzeugen eines Projekts in einer Initialisierungseinheit in eine Messdatengenerierungseinheit eingespeist werden, wobei ein Messdatensatz erzeugt wird, der einem mehrdimensionalen Abbild der Verletzung des Körperteils entspricht, die mit dem bildgebenden Verfahren ermittelt worden ist.

Im dritten Schritt kann aus dem Messdatensatz ein mehrdimensionales Modell des verletzten Körperteils in einem Modellgenerierungsmodul erzeugt werden.

Im vierten Schritt kann mit Hilfe des mehrdimensionalen Modells des verletzten Körperteils ein Modell des gesunden Körperteils in einem Modellvergleichsmodul erzeugt werden.

Nach einem Ausführungsbeispiel kann das mehrdimensionale Modell des Implantats an die Belastungsverhältnisse, denen der gesunde Körperteil ausgesetzt ist, angepasst werden.

Insbesondere kann das modifizierte mehrdimensionale Modell des Implantats Hohlräume oder poröse Abschnitte enthalten, welche das Verwachsen des Implantats mit dem umliegenden Gewebe fördern.

Nach einem Ausführungsbeispiel entsprechen die Abmessungen des Implantats den Abmessungen eines durch die Verletzung des Körperteils entstandenen Hohlraums, sodass das Implantat zumindest ein Volumen einnimmt, welches dem Volumen des Hohlraums entspricht.

Insbesondere kann das additive Herstellungsverfahren ein Lasersinterverfahren oder ein Laserschmelzverfahren oder ein Elektronenstrahlschmelzverfahren (EBM) umfassen. Ebenso können Extrusionsverfahren oder 3D Druckverfahren zur Anwendung kommen, welche auch als Binder in Bett Verfahren bekannt sind. Der Rohstoff für das Implantat kann zumindest ein Element aus der Gruppe der Metalle, der keramischen Materialien oder der Kunststoffe umfassen. Insbesondere kann der Rohstoff für das Implantat Titan enthalten. Der Rohstoff für das Implantat kann pulverförmig, flüssig oder als Filament vorliegend sein.

Nach einem Ausführungsbeispiel kann der Messdatensatz, der einem mehrdimensionalen Abbild der Verletzung des Körperteils entspricht, durch einen Algorithmus in das mehrdimensionale Modell der Verletzung des Körperteils transformiert werden. Nach einem Ausführungsbeispiel kann das mehrdimensionale Modell der Verletzung des Körperteils durch einen Algorithmus in das mehrdimensionale Modell des gesunden Körperteils transformiert werden. Insbesondere kann ein Messdatensatz eines gesunden Körperteils des Patienten generiert werden. Diese Vorgehensweise eignet sich insbesondere dann, wenn es sich bei dem verletzten Körperteil um einen Körperteil handelt, für den ein spiegelbildlich angeordneter Körperteil im oder am Körper des Patienten vorhanden ist, beispielsweise ein Bestandteil des Kopfs, Extremitäten, insbesondere ein Bestandteil der Arme oder Beine. Ein derartiger Körperteil wird in der Folge als chirale Modifikation des verletzten Körperteils bezeichnet, wobei unter chiraler Modifikation eine im Spiegelbild deckungsgleiche Anordnung zu verstehen ist. Eine linke Hand ist beispielsweise eine chirale Modifikation einer rechten Hand. Wenn ein Körperteil von der Verletzung betroffen ist, von welchem eine chirale Modifikation im oder am Körper existiert, kann dieser Körperteil durch eine Spiegelung in ein Abbild des verletzten Körperteils im unverletzten Zustand, also im gesunden Zustand, überführt werden. Nach diesem Ausführungsbeispiel liegen dem Projektmitarbeiter ein mehrdimensionales Modell des verletzten Körperteils sowie ein mehrdimensionales Modell des gesunden Körperteils vor, der durch Spiegelung seiner chiralen Modifikation erhalten worden ist. Das heisst, der Projektmitarbeiter kann eine Visualisierung des verletzten Körperteils und eine Visualisierung des gesunden Körperteils vornehmen.

Nach einem Ausführungsbeispiel kann im siebenten Schritt das modifizierte mehrdimensionale Modell des Implantats mittels eines Druckdateierstellungsmoduls in eine Druckdatei für ein additives Herstellungsverfahren umgewandelt werden. Insbesondere können dabei bestehende Standardformate für additive Herstellungsverfahren wie STL, 3MF oder AMF ausgelassen werden und direkt die additiven Herstellungsverfahren angesteuert werden. Dabei werden die spezifischen Prozessführungsparameter der additiven Herstellungsverfahren direkt im Druckdateierstellungsmodul definiert und als lesbare Datei an die Steuerung des additiven Herstellungsverfahrens übergeben.

Nach einem Ausführungsbeispiel kann im achten Schritt mittels eines Anweisungsgenerierungsmoduls aus der Druckdatei Anweisungen zur schichtweisen Herstellung des Implantats durch ein additives Herstellungsverfahren in der additiven Herstellungsvorrichtung erstellt werden. Die Herstellung kann an der Klinik selber oder bei einem kliniknahen Dienstleister erfolgen.

Nach einem Ausführungsbeispiel kann die Druckdatei für das additive Herstellungsverfahren einer additiven Herstellungsvorrichtung über ein Netzwerk, beispielsweise unter Nutzung des Internets an die additive Herstellungsvorrichtung übermittelt werden.

Nach einem Ausführungsbeispiel kann das bildgebende Verfahren zumindest ein Verfahren aus der Gruppe der Röntgenverfahren, MRI-Verfahren, CT-Verfahren, Ultraschallverfahren, optischen Scanverfahren oder Laserscanverfahren umfassen.

Ein System zur Herstellung eines Implantats umfasst eine Initialisierungseinheit zur Erzeugung eines Projekts. Das System umfasst eine Messdatengenerierungseinheit, mittels welcher ein Messdatensatz aus einer Messung am Patienten mittels eines bildgebenden Verfahrens generierbar ist, wobei der Messdatensatz ein Abbild eines verletzten Körperteils enthält, welches mittels eines bildgebenden Messverfahrens erstellt ist. Das System umfasst des Weiteren ein Modellgenerierungsmodul, mittels welchem ein mehrdimensionales Modell des verletzten Knoches aus dem Messdatensatz generierbar ist. Gegebenenfalls ist ein Modellvergleichsmodul vorgesehen, mittels welchem ein mehrdimensionales Modell eines gesunden Körperteils generierbar ist. Mittels einem Implantatauswahlmodul kann ein Implantat aus einer Bibliothek von Implantatmodellen derart auswählbar sein, dass das Implantatmodell mittels des Modellgenerierungsmoduls oder des Modellvergleichsmoduls an das mehrdimensionale Modell des verletzten oder gesunden Körperteils anpassbar ist und/oder mittels einem Implantatgenerierungsmodul, mittels welchem ein mehrdimensionales Modell eines Implantats derart erzeugbar ist, dass ein für die spezifische Verletzung neu entwickeltes mehrdimensionales Implantatmodell generierbar ist, sodass mit zumindest einem der Implantatauswahlmodule oder Implantatgenerierungsmodule ein modifiziertes mehrdimensionales Modell des Implantats erhältlich ist. Das Implantatmodell kann in ein Druckdateierstellungsmodul für ein additives Herstellungsverfahren aufnehmbar sein. Ein Anweisungsgenerierungsmodul kann vorgesehen sein, mittels welchem eine vom Druckdateierstellungsmodul erstellte Druckdatei in eine Herstellungsanweisung für eine additive Herstellungsvorrichtung umwandelbar ist. Die additive Herstellungsvorrichtung ist vorgesehen, um das Implantat gemäss der Herstellungsanweisung zu erzeugen oder herzustellen. Aus dem modifizierten mehrdimensionalen Modell des Implantats ist mit zumindest einem der Implantatauswahlmodule oder Implantatgenerierungsmodule ein patientenspezifisches Implantat erhältlich.

Nach einem Ausführungsbeispiel kann mittels dem Implantatauswahlmodul oder dem Implantatgenerierungsmodul zumindest eines der mehrdimensionalen Modelle des verletzten Körperteils, des gesunden Körperteils oder des Implantats in der Perspektive des Betrachters in einer zweidimensionalen Bildebene mittels eines Implantatbearbeitungsmoduls manipulierbar sein. Insbesondere kann das Implantatbearbeitungsmodul zumindest ein Element aus der Gruppe der Trennmodule, der Schneidmodule, der Verschiebemodule oder der Rotationsmodule enthalten.

Nach einem Ausführungsbeispiel kann das mittels des Implantatauswahlmoduls ausgewählte Implantatmodell oder das mit dem Implantatgenerierungsmodul entwickelte Implantatmodell mittels eines Belastungsintensitätsmoduls auf das Vorhandensein von übermässigen Belastungen für den Körperteil überprüfbar sein.

Insbesondere kann das mehrdimensionale Modell des Implantats vom Projektmitarbeiter aus dem mehrdimensionalen Modell der Verletzung des Körperteils oder des gesunden Körperteils erstellt werden. Bei Erstellung des Modells können Spannungszustände im Implantat über eine Finite-Elemente-Methode bestimmt werden. Beispielsweise kann der Gewebeaufbau in der Umgebung des Defekts Rückschlüsse über die örtliche Belastung des Gewebes an der Begrenzungsoberfläche des Defekts liefern. Diese örtliche Belastung muss durch das Implantat aufgenommen werden können. WO2013091085 beschreibt die Verwendung der Finite Elemente Methode (FEM) zur Generierung eines Modells eines porösen Implantats mit lokal unterschiedlichen mechanischen Eigenschaften. Diese Eigenschaften werden in Abhängigkeit von den Spannungen ermittelt, die auf das Implantat wirken können.

Ein Belastungsprofil (also die Zugspannungen, Druckspannungen, Schubspannungen) des Körperteils, insbesondere des Knochens können aus der Knochendichte gemäss einem von Pal et al. aufgestellten empirischen Zusammenhang ermittelt werden. (siehe auch Pal, B., Gupta, S., New, A., 2009, "A Numerical Study of Failure Mechanisms in the Cemented Resurfaced Femur: Effects of Interface Characteristics and Bone Remodelling", Proceedings of the Institution of Mechanical Engineers, Part H: Journal of Engineering in Medicine, 223(4), 471 -484).

Dieses Belastungsprofil des Körperteils, also insbesondere des Knochens kann als Randbedingung für die Erstellung des FEM-Modells des Implantats dienen, sodass das FEM-Modell das Belastungsprofil berücksichtigen kann. Die Dichte des Knochens kann aus dem Messdatensatz, welche das bildgebende Verfahren geliefert hat, für jeden Punkt der Begrenzungsoberfläche ermittelt werden. Daher ist unter Nutzung dieser Informationen aus dem bildgebenden Verfahren eine Modellgeometrie erhältlich, welche dieses Belastungsprofil berücksichtigt. Insbesondere kann das Belastungsprofil auch Rückschlüsse auf die dynamische Belastung des Knochens und in der Folge des Implantats liefern, sodass bei der Erstellung eines mehrdimensionalen Modells des Implantats ein dynamischer Belastungszustand berücksichtigt werden kann und Beschädigungen des Implantats oder des Knochens durch die dynamische Belastung vermieden werden können.

Das Verfahren oder System nach einem der vorhergehenden Ausführungsbeispiele kann zur Herstellung eines Implantats aus der Gruppe der Spinalimplantate, Dentalimplantate, orthopädischen Implantate, künstlichen Linsen, Knochenteile, Knorpeltele, Augenimplantate, Gelenkimplantate, kosmetische Implantate verwendet werden.

Nach einem Ausführungsbeispiel kann das patientenspezifische Implantat innerhalb von einer Woche nach dem Start der Initialisierungseinheit erhältlich sein, insbesondere kann bei lokaler Verfügbarkeit der additiven Herstellungsvorrichtung ein patientenspezifisches Implantat innerhalb eines Zeitraums von maximal drei Tagen erhältlich sein.

Die Erstellung des mehrdimensionalen Modells und die Anpassung desselben durch den Projektmitarbeiter kann sehr einfach in wenigen Arbeitsschritten am Arbeitsplatz des Projektmitarbeiters vorgenommen werden. Der Projektmitarbeiter benötigt hierzu einen Rechner mit Internetzugang, ein Eingabeelement, einen Bildschirm zur Visualisierung des mehrdimensionalen Modells. Der Projektmitarbeiter kann sich mit einem handelsüblichen Browserprogramm Zugang zu einer Webseite verschaffen, welche die Modellierungssoftware zur Erstellung des mehrdimensionalen Modells des verletzten Körperteils aus dem Datensatz enthält. Die Software umfasst vorteilhafterweise auch Zugang zu einer Datenbank, welche die Verletzung eindeutig einem Patienten zuordnen kann. Der Projektmitarbeiter kann in einem ersten Schritt einen Patientendatensatz erzeugen. Nach Erstellung des Patientendatensatzes lädt der Projektmitarbeiter den Messdatensatz in die Datenbank aufgrund der Anweisungen, die ihm auf dem Bildschirm angezeigt werden. Der Messdatensatz ist eine Datei, die von der Vorrichtung zur Durchführung des bildgebenden Verfahrens erzeugt worden ist. Diese Datei kann von einem beliebigen Datenträger, beispielsweise von einer CD-ROM, einem USB-Stick oder dergleichen stammen. Die Datei kann insbesondere eine Bilddatendatei umfassen, beispielsweise eine bitmap - Datei, eine Portable Network Grafics (PNG) Datei, eine jpg-Datei, eine Datei, welche die Originaldaten des bildgebenden Verfahrens insbesondere gemäss des DICOM Standards enthält, oder ein PDF-Dokument umfassen. In Abhängigkeit von der Geschwindigkeit des Internetzugangs und der Grösse der Datei kann dieser Vorgang des Einlesens des Messdatensatzes in die Modellierungssoftware in weniger als 10 min abgeschlossen sein, wenn die Übertragungsgeschwindigkeit einige Mbit/sec beträgt.

Nach dem Laden der Messdatendatei wird durch die Modellierungssoftware ein mehrdimensionales Modell des oder der verletzten Körperteile erstellt. Dieses mehrdimensionale Modell ist insbesondere als ein Oberflächenmodell ausgebildet, mittels welchem der verletzte Körperteil in der Lage angezeigt wird, welche der verletzte Körperteil bei Durchführung des bildgebenden Verfahrens hatte. Der Projektmitarbeiter findet somit auf dem Bildschirm die Ansicht vor, die der Ansicht entspricht, welche der verletzte Körperteil zum Zeitpunkt der Durchführung des bildgebenden Verfahrens gehabt hat.

Der Projektmitarbeiter kann die Lage des mehrdimensionalen Modells des Körperteils am Bildschirm derart verändern, dass ihm die gewünschte Ansicht am Bildschirm angezeigt wird. Der Projektmitarbeiter kann die Lage von Teilen des mehrdimensionalen Modells des Körperteils am Bildschirm derart verändern, dass sie in der gewünschten Lage zueinander liegen.

Insbesondere kann der Projektmitarbeiter das mehrdimensionale Modell des Körperteils frei in jede beliebige Richtung drehen oder in jede beliebige Richtung verschieben. Hierzu wählt der Projektmitarbeiter mit einem Eingabeelement einen Punkt des mehrdimensionalen Modells aus. Das Eingabeelement kann ein akustisches Eingabeelement, beispielsweise ein Mikrophon, ein berührungssensitives Eingabeelement, beispielsweise einen Touchscreen, der gegebenenfalls eine Multitouchfunktion enthält, einen Joystick, ein Gamepad, ein Stift oder ein optisches Eingabeelement umfassen, beispielsweise einen Scanner, ein Stift mit Scanfunktion, oder ein Eingabeelement welches eine Kombination von mindestens zwei der akustischen, berührungssensitiven, optischen Eingabeelemente umfasst. Insbesondere kann mit dem Eingabeelement Gestik, beispielsweise Fingerbewegungen erfassbar und in Eingabeanweisungen umwandelbar sein. Der vom Eingabeelement ausgewählte Punkt ist im mehrdimensionalen Modell in Bezug auf jeden weiteren Punkt des mehrdimensionalen Modells festgelegt, da der Punkt zu jedem anderen Punkt des dreidimensionalen Modells einen eindeutig bestimmten Abstand sowie eine eindeutig bestimmte Raumrichtung aufweist. Das mehrdimensionale Modell kann hierbei aus einem dreidimensionalen Netz von Punkten bestehen. Durch Verbinden der Punkte mit Dreiecken kann die Oberfläche des Modells dargestellt werden Der ausgewählte Punkt kann vom Projektmitarbeiter mittels des Eingabeelements auf dem Bildschirm bewegt werden. Die Bewegung kann eine Verschiebung in Bezug auf einen Referenzpunkt sein, wobei der Referenzpunkt beispielsweise der Ursprung eines Koordinatensystems ist. Das mehrdimensionale Modell kann eine Mehrzahl von Modellfragmenten umfassen. Möchte der Projektmitarbeiter eines der Modellfragmente relativ zu einem anderen Modellfragment verschieben, wird zuerst durch die Lage des mehrdimensionalen Modells in Bezug auf die Bildschirmebene eine Betrachtungsperspektive erzeugt. Der Projektmitarbeiter sieht das mehrdimensionale Modell aus der Betrachterperspektive als Projektion auf die Bildschirmebene. Der Projektmitarbeiter kann ein Modellfragment relativ zu einem weiteren Modellfragment verschieben, indem er auf dem Bildschirm die Umrisse des zu verschiebenden Modellfragments auswählt und den gewünschten Abstand sowie die Richtung der Verschiebung, das heisst den Neigungswinkel in Bezug auf eine horizontale (oder vertikale) Bildschirmreferenzlinie festlegt.

Die Bewegung kann eine Drehbewegung sein, wobei die Drehbewegung in Bezug auf eine Drehachse, die senkrecht zur Betrachtungsrichtung liegt, erfolgt. Das heisst, die Drehachse verläuft normal zur Bildschirmebene. Die Position der Drehachse kann der Projektmitarbeiter festlegen, indem er einen Punkt der gewünschten Drehachse auf dem Bildschirm auswählt. Die Position der Drehachse kann vom System auch automatisch ins Zentrum des Fragments gelegt werden, beispielsweise in den Masseschwerpunkt oder in das Zentrum eines umschliessenden Volumenkörpers. Den gewünschten Drehwinkel kann der Projektmitarbeiter durch Festlegen eines Drehwinkels auswählen.

Der Projektmitarbeiter kann auch einen Schnitt durch das mehrdimensionale Modell erzeugen, indem er eine Schnittebene definiert. Durch das Definieren der Schnittebene erfolgt ein Durchtrennen des mehrdimensionalen Modells entlang dieser Schnittebene. Die Schnittebene kann man sich als virtuelles Messer vorstellen, mittels welchem das mehrdimensionale Modell in Richtung der Betrachtungsperspektive durchtrennt wird. Das heisst, die Schnittebene liegt normal zur Bildschirmoberfläche. Der Projektmitarbeiter kann jede beliebige Schnittebene erzeugen, indem er zwei Punkte auf der Bildschirmoberfläche auswählt, welche die Schnittgerade der Schnittebene mit der Bildschirmoberfläche erzeugen. Alternativ kann der Projektmitarbeiter einen Punkt anwählen und auf dem Bildschirm mit dem Eingabeelement eine Strecke definieren, welche die Richtung der Schnittebene festlegt. Dabei bildet der Eingabepunkt, der mit dem Eingabeelement auf dem Bildschirm erzeugt wird, die Spitze des virtuellen Messers aus. Durch Bewegung des Eingabeelements wird eine Schnittlinie, bestehend aus mehreren kleinen Schnittlinien (-ebenen), definiert, die insbesondere eine horizontale oder vertikale Schnittlinie sein kann oder auch in einem beliebigen Neigungswinkel zu der horizontalen Schnittlinie angeordnet sein kann. Horizontal und vertikal bezieht sich hierbei auf die Position der Schnittlinie in der Bildschirmebene.

Das mehrdimensionale Modell kann somit durch eine Verschiebung oder Drehung bzw. Rotation beliebig im Modellraum ausgerichtet werden, um dann durch eine benutzerdefinierte Schnittlinie durchtrennt zu werden.

Die Manipulationen des Durchtrennens oder Schneidens, des Verschiebens oder des Rotierens erfolgen nur aus der Perspektive des Betrachters des Bildschirms in der jeweiligen 2D-Bild-Ebene, die durch den Bildschirm gebildet wird. Dadurch kann die Bedienkomplexität reduziert werden, sodass eine einfache und intuitive Manipulation von mehrdimensionalen Modellen ohne vorgängige Schulung möglich ist. Dieses Verfahren eignet sich aufgrund der Vereinfachung auch für die Ausführung auf Tablets.

Alternativ ist es möglich, mittels eines Positionierbefehls eine automatische Zuordnung der Oberflächen von Körperteilfragmenten zueinander zu erstellen und die Verbindungsflächen zwischen den neu positionierten Körperteilen oder Körperteilfragmenten zu erstellen.

Der Projektmitarbeiter kann anhand der visuellen Darstellung der Anordnung der Körperteilfragmente feststellen, ob die vorgeschlagene Anordnung Fehler enthält.

Beispielsweise kann er das mehrdimensionale Modell eines gesunden Körperteils mit dem mehrdimensionalen Modell des verletzten Körperteils überlagern. Bei dieser Überlagerung treten am Ort der Verletzung Abweichungen zwischen den beiden mehrdimensionalen Modellen auf. Diese Abweichungen können beispielsweise durch eine besondere Farbgebung, durch eine andere Linienart oder Linienstärke, durch einen Warnhinweis, wie beispielsweise ein Blinken sichtbar gemacht werden. Derartige Abweichungen kann der Projektmitarbeiter mittels eines manuellen Korrekturbefehls korrigieren. Er kann beispielsweise die Lage der Körperteilfragmente zueinander ändern, indem er den Winkel der Körperteilfragmente zueinander oder den Abstand der Körperteilfragmente voneinander verändert.

Wenn das mehrdimensionale Modell des Körperteils in der gewünschten Lage auf dem Bildschirm angezeigt wird, kann der Projektmitarbeiter ein mehrdimensionales Modell des Implantats erstellen.

Der Projektmitarbeiter kann beispielsweise das gewünschte Implantat aus einem Satz von kommerziell erhältlichen Implantaten auswählen. Hierzu bedient sich der Projektmitarbeiter einer Bibliothek, welche Abbildungen dieser kommerziell erhältlichen Implantate enthält. Aus dieser Bibliothek wählt der Projektmitarbeiter ein derartiges Standardimplantat aus und positioniert es in der gewünschten Lage, sodass der verletzte Körperteil durch das Standardimplantat in der Lage fixiert werden kann, die eine Heilung der Verletzung des Körperteils ermöglicht.

Gemäss eines Ausführungsbeispiels sind derartige Standardimplantate als Standardplatten ausgebildet. Standardplatten zum Fixieren von Körperteilfragmenten werden von den Herstellern meistens in flacher Form geliefert. Um sie am Patienten zu verwenden, müssen die Platten vor oder während der Operation angepasst, d.h. gebogen werden, um an die individuellen anatomischen Körperteilform des Patienten zu passen (z.B. insbesondere im Gesicht).

Dem Projektmitarbeiter wird eine skalierbare Abbildung der möglichen Standardimplantate zur Auswahl des Standardimplantats angezeigt und er erhält somit eine virtuelle Vorschau, sodass er präoperativ planen kann, ob ein derartiges Standardimplantat direkt einsetzbar ist, oder ob es einer Modifikation des Standardimplantats bedarf. Daher kann der Projektmitarbeiter festlegen, welche Grösse und/oder Form des Standardimplantats oder eines modifizierten Implantats für den operativen Eingriff verwendet werden kann. Hierzu kann er das Baukastensystem benutzen. Da jedes der Modulelemente mit einem oder mehreren definierten Belastungszuständen verknüpft ist, wird bei Auswahl des Modulelements automatisch geprüft, ob das gewählte Modulelement für das modifizierte dreidimensionale Modell des Implantats geeignet ist. Der Projektmitarbeiter erhält eine Rückmeldung, wenn das Modulelement nicht für die gewünschte Belastungssituation im Körper geeignet ist.

Zusätzlich zu der Bibliothek von Standardimplantaten wird dem Projektmitarbeiter das mehrdimensionale Modell des verletzten und/oder gesunden Körperteils angezeigt. Der Projektmitarbeiter kann die Abbildung des Standardimplantats seiner Wahl auswählen und auf eine beliebige Stelle des Körperteils ziehen. Die Abbildung des Standardimplantats enthält ein mehrdimensionales Modell des Standardimplantats. Dieses mehrdimensionale Modell des Standardimplantats kann mittels eines Algorithmus derart verändert werden, dass die Krümmung des mehrdimensionalen Modells des Standardimplantats mit der Krümmung des Körperteils in Übereinstimmung gebracht wird. Diese Übereinstimmung erfolgt beispielsweise nach dem Verfahren des Spline-Fittings. Das entsprechend angepasste Implantat kann als mehrdimensionales Modell auf dem mehrdimensionalen Modell des Körperteils farblich unterscheidbar angezeigt werden. Mit virtuellen Greifern können Position und Winkel des Implantats weiter angepasst werden. Insbesondere kann das mehrdimensionale Modell des Implantats auf der Oberfläche des mehrdimensionalen Modells des Körperteils verschoben werden. Das mehrdimensionale Modell des Implantats kann auch durch eine Rotationsbewegung in seiner Lage in Bezug auf die Oberfläche des mehrdimensionalen Modells des Körperteils verändert werden, wodurch die Formgebung des Implantats angepasst wird, um eine optimale Anbindung an den Körperteil zu ermöglichen. Beispielsweise kann nach einer Fraktur eines Knochens ein Plattenelement eingesetzt werden, welches die Knochenteile zueinander in der Position fixiert, die es erlauben, den Knochen in dem Zustand wieder herzustellen, in welchem er sich vor der Fraktur befunden hat. Da jedes der Modulelemente in seinen Festigkeitseigenschaften definiert ist, kann aus der Summe der Modulelemente die Festigkeit des gewünschten Implantats mittels des modifizierten mehrdimensionalen Modells des Implantats ermittelt werden.

Um das Implantat am Körperteil dauerhaft zu befestigen, bedarf es eines gegebenenfalls eines Fixierungselements, durch welches die Verbindung zwischen dem Körperteil und dem Implantat geschaffen wird, um das Implantat in der gewünschten Position am Körperteil anzubringen sowie dauerhaft zu befestigen. Das Fixierungselement kann Fixierungsmittel umfassen, beispielsweise Schrauben, welche eine Befestigung der des Implantats am Knochen oder den Knochenfragmenten erlauben. Der Projektmitarbeiter kann die Position der Fixierungsmittel frei festlegen, er kann beispielsweise deren Lage, Anzahl und/oder Winkel festlegen. Der Projektmitarbeiter kann ein Fixierungsmittel aus einer Datenbank, welche sämtliche kommerziell verfügbaren Fixierungsmittel enthält, auswählen. Auch die Fixierungsmittel sind Ausführungsbeispiele für Modulelemente.

Der Projektmitarbeiter kann beliebige Ansichten des mehrdimensionalen Modells generieren, sodass er das Fixierungselement und die Fixierungsmittel von allen Seiten betrachten kann und den operativen Eingriff visualisieren kann, sodass er die optimale Form des Fixierungselements sowie die optimale Anordnung der Fixierungsmittel ermitteln kann. Insbesondere kann die Dichte des Knochens an der gewünschten Position des Fixierungselements aus dem Messdatensatz ermittelt werden. Die Knochendichte kann in Beziehung zur Festigkeit des Knochens gesetzt werden. In Kenntnis der Knochendichteverteilung kann somit die optimale Position des Fixierungsmittels am Knochen gefunden werden. Des Weiteren kann die maximale Belastung des Knochens an der gewünschten Position des Fixierungsmittels bestimmt werden, sodass der Projektmitarbeiter unmittelbar eine Rückmeldung erhält, ob das Fixierungsmittel in der gewünschten Position seinen Zweck erfüllen kann. Des Weiteren kann in der näheren Umgebung der gewünschten Position des Fixierungsmittels nach einer alternativen Position gesucht werden, wobei die alternative Position zumindest dadurch gekennzeichnet ist, dass die Belastungsobergrenze für das Fixierungsmittel höher ist, als in der vorherigen manuell definierten Position. Die Knochendichteverteilung kann farblich gekennzeichnet werden, beispielsweise mittels eines Farbverlaufs aus Falschfarben, sodass ein schnelles Auffinden der optimalen Position und/oder Dimension des Fixierungsmittels erleichtert wird. In gleicher Weise kann die Einbaurichtung des Fixierungsmittels optimiert werden. Eine derartige farbliche Kennzeichnung dient als Entscheidungshilfe für das Festlegen der Positionen und/oder Dimensionen des/der Fixierungsmittel.

Somit kann der Projektmitarbeiter die Position oder Dimension der Fixierungsmittel auf dem mehrdimensionalen Modell des Implantats gleichzeitig mit der Position auf dem mehrdimensionalen Modell des Körperteils bestimmen. Des Weiteren kann er Verbindungselemente zwischen je zwei Fixierungsmitteln als mehrdimensionales Modell eines patientenspezifischen Implantats erzeugen. Die Verbindungselemente erstrecken sich zwischen Aufnahmeöffnungen für die Fixierungsmittel. Die Verbindungselemente und Aufnahmeöffnungen können in Bezug auf ihre Formstabilität bei unterschiedlichen Lastzuständen, sowie in Bezug auf ihre Festigkeit optimiert werden.

Die Formstabilität kann auch durch eine Dimension, insbesondere die Wandstärke der Verbindungselemente oder gegebenenfalls eines die Verbindungselemente einschliessenden Plattenelements verändert werden. Somit kann der Projektmitarbeiter die Wandstärke der Verbindungselemente sowie die Wandstärke des Plattenelements anpassen und Verbindungselemente durch Plattenelemente ersetzen oder umgekehrt Plattenelemente durch Verbindungselemente zu ersetzen sowie Verbindungselemente hinzufügen oder entfernen.

Des Weiteren kann der Projektmitarbeiter die Eindringtiefe des Fixierungsmittels im mehrdimensionalen Modell des Körperteils variieren. Hierzu kann das mehrdimensionale Modell des Körperteils nach Art eines Röntgenbilds teilweise transparent dargestellt werden. Der Grad der Transparenz und/oder Grauwert kann proportional zur Körperteildichte, insbesondere der Knochendichte an der entsprechenden Stelle sein. Somit kann die optimale Länge des Fixierungsmittels ermittelt werden, um bestmöglichen Halt im Körperteil, insbesondere im Knochen, zu gewährleisten. Die Ermittlung der Länge eines Fixierungsmittels kann automatisch unter Berücksichtigung der Knochendichteverteilung erfolgen. Das gewünschte Fixierungsmittel kann mit den in der Bibliothek gespeicherten Fixierungsmitteln abgeglichen werden, sodass der Projektmitarbeiter entweder ein gespeichertes Fixierungsmittel wählen kann oder ein patientenspezifisches Fixierungsmittel entwerfen kann.

Insbesondere kann aus dem Messdatensatz, also beispielweise aus Tomographiebildern für die jeweilige Position, Dimension und Ausrichtung des Fixierungsmittels ein Schichtbild entlang der Längsachse des Fixierungsmittels errechnet und dargestellt werden. Diesem Schichtbildausschnitt kann ein massstabsgetreues mehrdimensionales Modell oder dessen 2D-Umriss aus beliebiger Betrachtungsperspektive des ausgewählten Fixierungsmittels überlagert werden. Die Dimension, beispielsweise die Länge und/oder die Ausrichtung des Fixierungsmittels kann beliebig variiert werden. Der Projektmitarbeiter sieht zu jedem Zeitpunkt, welche anatomischen Strukturen im und um den betroffenen Körperteil, insbesondere dem Knochen, berührt oder tangiert werden. So kann er diese Parameter optimal planen. Auch die Position des Fixierungsmittels kann bei Bedarf nochmals angepasst werden, falls sich aufgrund der Visualisierung zeigt, dass die gewählte Position anatomisch ungünstig ist.

Die für die Modellierung zum Einsatz kommende Software verfügt über integrierte Designrichtlinien, um Stabilität und Herstellbarkeit des Implantats zu garantieren, welches das Ergebnis der Modellierung darstellt. Parameterkombinationen, welche zu einem instabilen oder nicht herstellbaren Implantat führen würden, werden von der Software verhindert und der Projektmitarbeiter kann bereits in der Modellierungsphase entsprechend gewarnt werden.

Versucht der Projektmitarbeiter einen Parameter auf einen Wert zu setzen oder eine Position zu definieren, welche dazu führen würde, dass das Implantat nicht mehr den Richtlinien entsprechen würde, erhält der Projektmitarbeiter sofort eine Rückmeldung, idealerweise sogar während der Eingabe des betreffenden Parameters. Eine visuelle Darstellung des Problems sowie Hinweise zur Lösung des Problems werden sofort angezeigt. Das ist ein entscheidender Usability-Vorteil, da Fehler unmittelbar, also somit ohne zeitliche Verzögerung, vermieden werden können. Die Eingaben des Projektmitarbeiters werden somit zu jedem Zeitpunkt überprüft, sodass der Arbeitsablauf zu jedem Zeitpunkt für den Projektmitarbeiter klar ist und ersichtlich ist, welche Schritte er als nächstes ausführen muss. Hierzu weist das Verfahren ein effizientes Kontrollsystem auf, mittels welchem die Benutzereingaben jederzeit überwacht werden können. Dieses Kontrollsystem kann beispielsweise regelbasiert sein. Mit dem Kontrollsystem kann zu jedem Zeitpunkt ein korrigierender Eingriff in den Verfahrensablauf vorgenommen werden.

Sämtliche Interaktionen des Benutzers können im Hintergrund aufgezeichnet werden. Diese aufgezeichneten Interaktionen können verwendet werden, um Modifikationen des Verfahrens automatisch zu testen oder den Verfahrensablauf zu verbessern, sodass die Qualität der sich aus der Interaktion des Benutzers während der Modellgenerierung ergebenden Implantate verbessert wird. Die historischen Planungsdaten können als Input verwendet werden, um Testdaten zum Entwickeln von (teil-)automatisierter Planungssoftware zu erhalten oder Daten als Input für Machine Learning Algorithmen (z.B. neuronale Netze) zum (teil-) automatisieren von Planungsschritten bereitzustellen.

Wenn dieser Arbeitsschritt im Verfahren abgeschlossen ist, kann der Schritt der Modellumwandlung erfolgen, indem das Implantat, sowie gegebenenfalls das Fixierungselement und das oder die Fixierungsmittel als mehrdimensionales Produktionsmodell erstellt werden. Hierzu wird das modifizierte mehrdimensionale Modell des Implantats in eine Druckdatei für ein additives Herstellungsverfahren umgewandelt, beispielsweise im STL oder CLI Format. Aus der Druckdatei können Anweisungen zur schichtweisen Herstellung des Implantats durch ein additives Herstellungsverfahren in einer additiven Herstellungsvorrichtung erstellt werden. Die Druckdatei kann an den Hersteller des Implantats sowie gegebenenfalls der Fixierungselemente sowie der Fixierungsmittel übermittelt werden, welcher die Herstellung des Implantats vornimmt. Diese Modellumwandlung kann auch bereits die Erstellung einer Druckerdatei umfassen. Mittels der Druckerdatei kann der Projektmitarbeiter das Implantat vor Ort herstellen, wenn er über eine Vorrichtung zur Durchführung eines additiven Herstellungsverfahrens verfügt. Daher ist der Projektmitarbeiter unmittelbar nach Durchführung des bildgebenden Verfahrens in der Lage, einen Herstellungsauftrag für das Implantat auszulösen und ein Implantat innerhalb von wenigen Stunden individuell zugeschnitten auf die Bedürfnisse des Patienten rechnergestützt zu erstellen. Das heisst, das Implantat steht dem Patienten innerhalb einiger Stunden bis maximal einiger Tage zur Verfügung, sodass die Wartezeit für den Patienten durch Anwendung des vorliegenden Verfahrens entscheidend verkürzt werden kann.

Ein Vorteil des Verfahrens und des Systems sowie des Computerprogrammprodukts nach einem der vorhergehenden Ausführungsbeispiele ist insbesondere die Bedienungsfreundlichkeit, die durch eine klare Benutzerführung bei der Modellierung erreicht wird, welche die komplexen geometrischen Zusammenhänge ohne Eingriff des Benutzers automatisch abwickelt. Demzufolge ist eine lange Einschulung des Benutzers zur Benutzung der Systemkomponenten überflüssig, weil insbesondere sämtliche geometrischen Zusammenhänge zwischen Körperteilmodell und Implantatmodell automatisiert im Hintergrund ohne Einwirkung des Benutzers angepasst werden können. Durch die in der zweidimensionalen Bildebene möglichen einfachen Manipulationen des Trennens, Verschiebens, Drehens können sämtliche möglichen Implantatpositionen oder Dimensionen vom Benutzer in Echtzeit ermittelt und überprüft werden, sodass dem Benutzer bereits das Resultat der optimierten Implantatposition bzw. Implantatdimension angezeigt wird. Das System ist mit einer einfachen Navigation ausgestattet, mit welcher jeder vorhergehende Schritt mit einem Befehl "Zurück" und jeder nachfolgende Schritt mit einem Befehl "Weiter" frei in der richtigen zeitlichen Abfolge angewählt werden kann. Jede Eingabe des Benutzers wird vom System aufgezeichnet und gespeichert. Das System ist vorzugsweise als webbasiertes System ausgebildet, welches dem Benutzer über ein Netzwerk bereitgestellt wird, sodass der Projektmitarbeiter über sämtliche Systemkomponenten verfügen kann, wenn er an das Netzwerk angeschlossen ist. Insbesondere kann das Netzwerk das Internet umfassen.

Die Aufgabe der Erfindung wird insbesondere durch ein Verfahren zur Herstellung eines patientenspezifischen Implantats gelöst, wobei eine Verletzung eines Körperteils eines Patienten mittels eines bildgebenden Verfahrens gemessen wird. Aus dieser Messung am Patienten wird ein Messdatensatz erzeugt, der einem mehrdimensionalen Abbild der Verletzung des Körperteils entspricht, der mit dem bildgebenden Verfahren ermittelt worden ist. Aus dem Messdatensatz wird ein mehrdimensionales Modell des verletzten Körperteils erzeugt. Mit Hilfe des mehrdimensionalen Modells des verletzten Körperteils kann ein Modell des gesunden Körperteils erzeugt werden, welches insbesondere die Kräfte enthält, welche durch eine Dauerbelastung auf dieses Körperteil wirken. Unter Dauerbelastung wird hierbei die Belastung verstanden, welche das Implantat für die gesamte Einsatzdauer ohne Schaden zu nehmen, ertragen muss, das heisst ohne zu versagen, beispielsweise ohne zu ermüden oder gar zu brechen.

In der Folge wird ein mehrdimensionales Modell des Implantats erstellt. Hierzu wird das mehrdimensionale Modell des Implantats an das mehrdimensionale Modell des Körperteils angepasst, sodass ein modifiziertes mehrdimensionales Implantatmodell erhalten wird. Das modifizierte mehrdimensionale Implantatmodell wird aus mindestens einem Modulelement generiert, wobei die Festigkeit des Modulelements so ausgewählt wird, dass sie mindestens der Festigkeit bei der maximalen Belastung des Körperteils entspricht, sodass die Belastung, der das Implantat im Gebrauchszustand maximal über seine gesamte Lebensdauer ausgesetzt ist, kleiner als die Dauerfestigkeit des Modulelements ist.

Die Dauerfestigkeit ist durch die maximal zulässigen Druck- Zug-, oder Biegespannungen bzw. Torsionsspannungen charakterisiert, die im Implantat auftreten können ohne dass das Implantat für die Dauer seines maximalen Einsatzes im Körper versagt, beispielsweise Mikrorisse auftreten oder sogar ein Bruch des Implantats erfolgt.

Nach einem Ausführungsbeispiel kann das modifizierte mehrdimensionale Implantatmodell eine Mehrzahl von Modulelementen enthalten.

Gegebenenfalls wird das mehrdimensionale Modell des Implantats in einem oder mehreren Iterationsschritten aufgrund einer Berechnung der Belastung des Körperteils derart verändert, dass das mehrdimensionale Modell des Implantats den Belastungen des Körperteils bestmöglich standhält und/oder die Befestigung des Implantats am Körperteil eine minimale Abnützung des Körperteils zur Folge hat.

Insbesondere kann ein für die vorliegende Verletzung speziell entwickeltes mehrdimensionales Implantatmodell aus dem mindestens einem Modulelement generiert werden.

Das modifizierte mehrdimensionale Implantatmodell kann mittels eines Implantatauswahlmoduls oder eines Implantatgenerierungsmoduls erzeugt werden. Wenn ein mehrdimensionales Modell eines Implantats mit einem Implantatgenerierungsmodul erzeugt wird, kann das mehrdimensionale Modell des Implantats an das mehrdimensionale Modell des verletzten oder gesunden Körperteils angepasst werden, sodass das modifizierte mehrdimensionale Modell des Implantats erhalten wird.

Mittels des Implantatauswahlmoduls kann ein Implantat aus einer Bibliothek von Implantatmodellen ausgewählt werden, wobei das ausgewählte Implantatmodell mittels des Modellgenerierungsmoduls oder des Modellvergleichsmoduls an das mehrdimensionale Modell des verletzten oder gesunden Körperteils angepasst wird.

Mittels des Implantatauswahlmoduls oder des Implantatgenerierungsmoduls kann das mehrdimensionale Implantatmodell derart erzeugt werden, dass ein für die vorliegende Verletzung speziell entwickeltes modifiziertes mehrdimensionales Implantatmodell aus dem mindestens einem Modulelement generierbar ist, sodass mit zumindest einem der Implantatauswahlmodule oder Implantatgenerierungsmodule das modifizierte mehrdimensionale Modell des Implantats erzeugt wird.

Nach einem Ausführungsbeispiel kann das modifizierte mehrdimensionale Modell des Implantats in eine Druckdatei für additives Herstellungsverfahren oder eine Herstellungsanweisung für ein subtraktives Herstellungsverfahren umgewandelt werden. Insbesondere kann die Druckdatei für den Betrieb in einer additiven Herstellungsvorrichtung verwendet werden. Die Druckdatei kann Anweisungen zur schichtweisen Erzeugung des Implantats enthalten. Mittels der Druckdatei können Anweisungen zur schichtweisen Herstellung des Implantats durch ein additives Herstellungsverfahren in einer additiven Herstellungsvorrichtung erstellt werden. Im Anschluss kann das Implantat in der additiven Herstellungsvorrichtung hergestellt werden. Insbesondere kann das Implantat schichtweise gemäss den in der Druckdatei für das additive Herstellungsverfahren bereitgestellten Anweisungen zur Herstellung des Implantats hergestellt werden.

Nach einem Ausführungsbeispiel kann zumindest eines der mehrdimensionalen Modelle des verletzten Körperteils, des gesunden Körperteils oder des Implantats in der Perspektive des Betrachters in einer zweidimensionalen Bildebene manipuliert oder angepasst werden. Insbesondere kann die Manipulation ein Durchtrennen oder Schneiden, ein Verschieben oder ein Rotieren zumindest eines der mehrdimensionalen Modelle des verletzten Körperteils, des gesunden Körperteils oder des Implantats umfassen. Insbesondere kann das mehrdimensionale Modell des Implantats eine Mehrzahl von Modulelementen enthalten, wobei die Modulelemente an das modifizierte mehrdimensionale Modell des verletzten Körperteils, des gesunden Körperteils oder des Implantats in der Perspektive des Betrachters in einer zweidimensionalen Bildebene angepasst werden können, wobei mittels der Modulelemente zumindest eine der Operationen eines Durchtrennens, Schneidens, eines Verschiebens, Zusammenfügens, Skalierens, Spiegelns, Zusammenschiebens, Aufklappens, Entfaltens oder einer Rotation erfolgen kann.

Insbesondere kann das Messergebnis nach Erzeugen eines Projekts in einer Initialisierungseinheit in eine Messdatengenerierungseinheit eingespeist werden, wobei ein Messdatensatz erzeugt wird, der einem mehrdimensionalen Abbild der Verletzung des Körperteils entspricht, die mit dem bildgebenden Verfahren ermittelt worden ist. Insbesondere kann aus dem Messdatensatz ein mehrdimensionales Modell des verletzten Körperteils in einem Modellgenerierungsmodul erzeugt werden.

Mit Hilfe des mehrdimensionalen Modells des verletzten Körperteils kann ein Modell des gesunden Körperteils in einem Modellvergleichsmodul erzeugt werden.

Nach einem Ausführungsbeispiel kann das mehrdimensionale Modell des Implantats an die Belastungsverhältnisse, denen der gesunde Körperteil ausgesetzt ist, angepasst werden.

Insbesondere kann das modifizierte mehrdimensionale Modell des Implantats Hohlräume oder poröse Abschnitte enthalten, welche das Verwachsen des Implantats mit dem umliegenden Gewebe fördern.

Nach einem Ausführungsbeispiel entsprechen die Abmessungen des Implantats den Abmessungen eines durch die Verletzung des Körperteils entstandenen Hohlraums, sodass das Implantat zumindest ein Volumen einnimmt, welches dem Volumen des Hohlraums entspricht.

Insbesondere kann das additive Herstellungsverfahren ein Lasersinterverfahren oder ein Laserschmelzverfahren oder ein Elektronenstrahlschmelzverfahren (EBM) umfassen. Ebenso können Extrusionsverfahren oder 3D Druckverfahren zur Anwendung kommen, welche auch als Binder in Bett Verfahren bekannt sind. Der Rohstoff für das Implantat kann zumindest ein Element aus der Gruppe der Metalle, der keramischen Materialien oder der Kunststoffe umfassen. Insbesondere kann der Rohstoff für das Implantat Titan enthalten. Der Rohstoff für das Implantat kann pulverförmig, flüssig oder als Filament vorliegen.

Nach einem Ausführungsbeispiel kann der Messdatensatz, der einem mehrdimensionalen Abbild der Verletzung des Körperteils entspricht, durch einen Algorithmus in das mehrdimensionale Modell der Verletzung des Körperteils transformiert werden. Nach einem Ausführungsbeispiel kann das mehrdimensionale Modell der Verletzung des Körperteils durch einen Algorithmus in das mehrdimensionale Modell des gesunden Körperteils transformiert werden. Insbesondere kann ein Messdatensatz eines gesunden Körperteils des Patienten generiert werden. Diese Vorgehensweise eignet sich insbesondere dann, wenn es sich bei dem verletzten Körperteil um einen Körperteil handelt, für den ein spiegelbildlich angeordneter Körperteil im oder am Körper des Patienten vorhanden ist, beispielsweise ein Bestandteil des Kopfs, Extremitäten, insbesondere ein Bestandteil der Arme oder Beine. Ein derartiger Körperteil wird in der Folge als chirale Modifikation des verletzten Körperteils bezeichnet, wobei unter chiraler Modifikation eine im Spiegelbild deckungsgleiche Anordnung zu verstehen ist. Eine linke Hand ist beispielsweise eine chirale Modifikation einer rechten Hand. Wenn ein Körperteil von der Verletzung betroffen ist, von welchem eine chirale Modifikation im oder am Körper existiert, kann dieser Körperteil durch eine Spiegelung in ein Abbild des verletzten Körperteils im unverletzten Zustand, also im gesunden Zustand, überführt werden. Nach diesem Ausführungsbeispiel liegen dem Projektmitarbeiter ein mehrdimensionales Modell des verletzten Körperteils sowie ein mehrdimensionales Modell des gesunden Körperteils vor, der durch Spiegelung seiner chiralen Modifikation erhalten worden ist. Das heisst, der Projektmitarbeiter kann eine Visualisierung des verletzten Körperteils und eine Visualisierung des gesunden Körperteils vornehmen.

Nach einem Ausführungsbeispiel kann das modifizierte mehrdimensionale Modell des Implantats mittels eines Druckdateierstellungsmoduls in eine Druckdatei für ein additives Herstellungsverfahren umgewandelt werden. Insbesondere können dabei bestehende Standardformate für additive Herstellungsverfahren wie STL, 3MF oder AMF ausgelassen werden und direkt die additiven Herstellungsverfahren angesteuert werden. Dabei werden die spezifischen Prozessführungsparameter der additiven Herstellungsverfahren direkt im Druckdateierstellungsmodul definiert und als lesbare Datei an die Steuerung des additiven Herstellungsverfahrens übergeben.

Nach einem Ausführungsbeispiel kann mittels eines Anweisungsgenerierungsmoduls aus der Druckdatei Anweisungen zur schichtweisen Herstellung des Implantats durch ein additives Herstellungsverfahren in der additiven Herstellungsvorrichtung erstellt werden. Die Herstellung kann an der Klinik selber oder bei einem kliniknahen Dienstleister erfolgen.

Nach einem Ausführungsbeispiel kann die Druckdatei für das additive Herstellungsverfahren einer additiven Herstellungsvorrichtung über ein Netzwerk, beispielsweise unter Nutzung des Internets an die additive Herstellungsvorrichtung übermittelt werden.

Nach einem Ausführungsbeispiel kann das bildgebende Verfahren zumindest ein Verfahren aus der Gruppe der Röntgenverfahren, MRI-Verfahren, CT-Verfahren, Ultraschallverfahren, optischen Scanverfahren oder Laserscanverfahren umfassen.

Ein System zur Herstellung eines Implantats umfasst eine Initialisierungseinheit zur Erzeugung eines Projekts. Das System umfasst eine Messdatengenerierungseinheit, mittels welcher ein Messdatensatz aus einer Messung am Patienten mittels eines bildgebenden Verfahrens generierbar ist, wobei der Messdatensatz ein Abbild eines verletzten Körperteils enthält, welches mittels eines bildgebenden Messverfahrens erstellt ist. Das System umfasst des Weiteren ein Modellgenerierungsmodul, mittels welchem ein mehrdimensionales Modell des verletzten Knoches aus dem Messdatensatz generierbar ist. Gegebenenfalls ist ein Modellvergleichsmodul vorgesehen, mittels welchem ein mehrdimensionales Modell eines gesunden Körperteils generierbar ist. Mittels eines Implantatauswahlmoduls kann ein Implantat aus einer Bibliothek von Implantatmodellen derart auswählbar sein, dass das Implantatmodell mittels des Modellgenerierungsmoduls oder des Modellvergleichsmoduls an das mehrdimensionale Modell des verletzten oder gesunden Körperteils anpassbar ist und/oder mittels einem Implantatgenerierungsmodul, mittels welchem ein mehrdimensionales Modell eines Implantats derart erzeugbar ist, dass ein für die spezifische Verletzung neu entwickeltes mehrdimensionales Implantatmodell aus mindestens einem Modulelement generierbar ist, sodass mit zumindest einem der Implantatauswahlmodule oder Implantatgenerierungsmodule ein modifiziertes mehrdimensionales Modell des Implantats erhältlich ist.

Das mehrdimensionale Modell des Implantats wird an das mehrdimensionale Modell des Körperteils angepasst, sodass das modifiziertes mehrdimensionales Modell des Implantats erhältlich ist, wobei das modifizierte mehrdimensionale Modell des Implantats aus dem mindestens einen Modulelement erzeugbar ist, wobei die Festigkeit des Modulelements auswählbar ist, dass sie mindestens der Festigkeit bei einer maximalen Belastung des Körperteils entspricht, sodass die Belastung, der das Implantat im Gebrauchszustand über seine gesamte Lebensdauer maximal ausgesetzt ist, kleiner als die Dauerfestigkeit des Modulelements ist.

Insbesondere kann das Modulelement eine bekannte Abmessungen sowie bei Auswahl eines Werkstoffs bekannte Festigkeitskennwerte aufweisen. Unter einer bekannten Abmessung wird eine definierte räumliche Ausdehnung verstanden, d.h. es handelt sich insbesondere um Längsabmessung, Breitenabmessung sowie Dicke. Das heisst, dass das Modulelement ein bestimmtes Volumen einnimmt und eine definierte Form aufweist. Insbesondere können zumindest ein erstes und ein zweites Modulelement vorgesehen sein. Die Abmessungen des ersten Modulelements können sich von den Abmessungen des zweiten Modulelements unterscheiden. Je zwei benachbarte Modulelemente können jeweils eine gemeinsame Passungsfläche aufweisen, wobei je zwei benachbarte erste und zweite Modulelemente an deren gemeinsamen Kante oder gemeinsamen Fläche für dieselbe Maximalbelastung ausgelegt sind.

Nach einem Ausführungsbeispiel können die Modulelemente als Bestandteil eines Baukastensystems ausgebildet sein. Dieses Baukastensystem kann eine Mehrzahl von Modulelementen mit Standardabmessungen enthalten oder Modulelemente, die zumindest eine gemeinsame Passungsfläche aufweisen. Die Modulelemente können miteinander kombiniert werden, beispielsweise über Nut-Feder Passungssysteme oder Schnappelemente oder Rastelemente. Mindestens je zwei Modulelemente können entlang einer gemeinsamen Passungsfläche derart zusammenfügbar sein, dass die über die Passungsfläche übertragene Belastung für beide Modulelemente übereinstimmt.

Das modifizierte mehrdimensionale Modell des Implantats, welches aus den Modulelementen erzeugt worden ist, kann in ein Druckdateierstellungsmodul für ein additives Herstellungsverfahren aufnehmbar sein. Nach einem Ausführungsbeispiel kann das aus den Modulelementen erzeugte modifizierte mehrdimensionale Modell des Implantats in ein Anweisungsmodul für eine Herstellungsanweisung für ein subtraktives Herstellungsverfahren umwandelbar sein. Ein Anweisungsgenerierungsmodul kann vorgesehen sein, mittels welchem eine vom Druckdateierstellungsmodul erstellte Druckdatei in eine Herstellungsanweisung für eine additive Herstellungsvorrichtung umwandelbar ist. Die additive Herstellungsvorrichtung ist vorgesehen, um das Implantat gemäss der Herstellungsanweisung zu erzeugen oder herzustellen. Aus dem modifizierten mehrdimensionalen Modell des Implantats ist mit zumindest einem der Implantatauswahlmodule oder Implantatgenerierungsmodule ein patientenspezifisches Implantat erhältlich.

Nach einem Ausführungsbeispiel kann mittels dem Implantatauswahlmodul oder dem Implantatgenerierungsmodul zumindest eines der mehrdimensionalen Modelle des verletzten Körperteils, des gesunden Körperteils oder des Implantats in der Perspektive des Betrachters in einer zweidimensionalen Bildebene mittels eines Implantatgenerierungsmoduls manipulierbar sein. Insbesondere kann das Implantatgenerierungsmodul zumindest ein Element aus der Gruppe der Trennmodule, der Schneidmodule, der Verschiebemodule, der Zusammenfügemodule, der Skaliermodule, der Spiegelmodule, der Module zum Zusammenschieben, der Module zum Aufklappen, der Module zum Entfalten oder der Rotationsmodule enthalten.

Ein Computerprogrammprodukt umfasst ein computerlesbares Medium, enthaltend Anweisungen, die, wenn sie auf einem Computer ausgeführt werden, ein dreidimensionales Modell eines Implantats erstellen können, wobei mittels der Anweisungen, wenn sie auf einem Computer ausgeführt werden, ein Messdatensatz einer Messdatengenerierungseinheit aus einer Messung am Patienten mittels eines bildgebenden Verfahrens generierbar ist, wobei der Messdatensatz ein Abbild eines verletzten Körperteils enthält, welches mittels eines bildgebenden Messverfahrens erstellt ist, wobei mittels der Anweisungen, wenn sie auf einem Computer ausgeführt werden, ein mehrdimensionales Modell des verletzten Knoches aus dem Messdatensatz in einem Modellgenerierungsmodul generierbar ist, wobei mittels der Anweisungen, wenn sie auf einem Computer ausgeführt werden, ein gegebenenfalls mehrdimensionales Modell eines gesunden Körperteils mittels eines Modellvergleichsmoduls generierbar ist, wobei mittels der Anweisungen, wenn sie auf einem Computer ausgeführt werden, ein Implantat aus einer Bibliothek von Implantatmodellen mittels eines Implantatauswahlmoduls derart auswählbar ist, dass das Implantatmodell mittels des Modellgenerierungsmoduls oder des Modellvergleichsmoduls an das mehrdimensionale Modell des verletzten oder gesunden Körperteils anpassbar ist und/oder wobei mittels einem Implantatgenerierungsmodul mittels der Anweisungen, wenn sie auf einem Computer ausgeführt werden, ein mehrdimensionales Modell eines Implantats derart erzeugbar ist, dass ein für die spezifische Verletzung neu entwickeltes mehrdimensionales Implantatmodell generierbar ist, sodass mit zumindest einem der Implantatauswahlmodule oder Implantatgenerierungsmodule ein modifiziertes mehrdimensionales Modell des Implantats erhältlich ist, wobei mittels der Anweisungen, wenn sie auf einem Computer ausgeführt werden, das modifizierte mehrdimensionale Modell des Implantats aus mindestens einem Modulelement erzeugbar ist, wobei die Festigkeit des Modulelements auswählbar ist, dass sie mindestens der Festigkeit bei einer maximalen Belastung des Körperteils entspricht, sodass die Belastung, der das Implantat im Gebrauchszustand über seine gesamte Lebensdauer maximal ausgesetzt ist, kleiner als die Dauerfestigkeit des Modulelements ist. Insbesondere kann das Implantatmodell in ein Druckdateierstellungsmodul für ein additives Herstellungsverfahren aufnehmbar sein oder in ein Anweisungsmodul für eine Herstellungsanweisung für ein subtraktives Herstellungsverfahren umwandelbar sein. Nach einem Ausführungsbeispiel kann ein Anweisungsgenerierungsmodul vorgesehen sein, sodass mittels der Anweisungen, wenn sie auf einem Computer ausgeführt werden, eine vom Druckdateierstellungsmodul erstellte Druckdatei in eine Herstellungsanweisung für eine additive Herstellungsvorrichtung umwandelbar ist. Durch zumindest eines der Implantatauswahlmodule oder Implantatgenerierungsmodule kann ein patientenspezifisches Implantat aus dem modifizierten mehrdimensionalen Modell des Implantats erhältlich sein. Das patientenspezifische Implantat kann aus einem Implantatmodell erhalten werden, welches aus mechanisch definierten Implantantauswahlmodulen oder Implantatgenerierungmodulen aufgebaut ist.

Nach einem Ausführungsbeispiel kann das mittels des Implantatauswahlmoduls ausgewählte modifizierte mehrdimensionale Modell des Implantats oder das mit dem Implantatgenerierungsmodul entwickelte modifizierte mehrdimensionale Modell des Implantats mittels eines Belastungsintensitätsmoduls auf das Vorhandensein von übermässigen Belastungen für das Implantat bzw. den das Implantat umgebenden Körperteil überprüfbar sein. Insbesondere kann die Dauerfestigkeit des mittels des Implantatauswahlmoduls ausgewählten modifizierten mehrdimensionalen Modells des Implantats oder des mit dem Implantatgenerierungsmoduls entwickelten modifizierten mehrdimensionalen Modells des Implantats aus der Dauerfestigkeit der Modulelemente ermittelbar sein und mittels des Belastungsintensitätsmoduls überprüfbar sein, ob die Dauerfestigkeit des modifizierten mehrdimensionalen Modells des Implantats für die Dauerbelastung des Implantats im Körperteil ausreichend ist.

Gemäss eines Ausführungsbeispiels sind derartige Standardimplantate als Standardplatten ausgebildet. Standardplatten zum Fixieren von Körperteilfragmenten werden von den Herstellern meistens in flacher Form geliefert. Um sie am Patienten zu verwenden, müssen die Platten vor oder während der Operation angepasst, d.h. gebogen werden, um an die individuellen anatomischen Körperteilform des Patienten zu passen (z.B. insbesondere im Gesicht). Eine Standardplatte kann ein Modulelement sein oder eines der Modulelemente umfassen.

Nachfolgend wird das erfindungsgemässe Verfahren anhand einiger Ausführungsbeispiele dargestellt. Es zeigen
Fig. 1 eine Darstellung der Verfahrensschritte gemäss eines ersten Ausführungsbeispiels der Erfindung,
Fig. 2 eine Darstellung des Verfahrensablaufs gemäss eines zweiten Ausführungsbeispiels der Erfindung.
Fig. 3 ein Detail der Generierung des modifizierten mehrdimensionalen Modell des Implantats

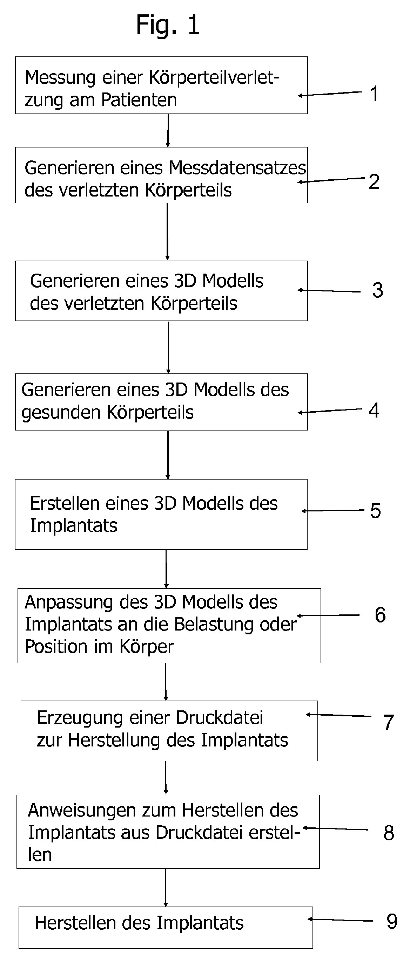

Das Verfahren zur Herstellung eines patientenspezifischen Implantats gemäss Fig. 1 umfasst die nachfolgend näher beschriebenen Verfahrensschritte. In einem ersten Schritt 1 wird eine Verletzung eines Körperteils eines Patienten mittels eines bildgebenden Verfahrens gemessen.

In einem zweiten Schritt 2 wird ein Messdatensatz erzeugt, der einem mehrdimensionalen Abbild der Verletzung des Körperteils entspricht, der mit dem bildgebenden Verfahren ermittelt worden ist. Mit anderen Worten wird im Schritt 2 ein Messdatensatz generiert, welcher die Bilddaten zur Erstellung eines mehrdimensionalen Modells der mit dem bildgebenden Verfahren festgestellten Verletzung des Körperteils dient.

In einem dritten Schritt 3 wird aus dem Messdatensatz ein mehrdimensionales Modell des verletzten Körperteils erzeugt. Mit dem Messdatensatz kann mit anderen Worten ein dreidimensionales Modell generiert werden, welches insbesondere die Verletzung des Körperteils zeigt. Nach einem Ausführungsbeispiel kann der Datensatz, der einem mehrdimensionalen Abbild verletzten Körperteils entspricht, durch einen Algorithmus in das mehrdimensionale digitale Modell des verletzten Körperteils transformiert werden.

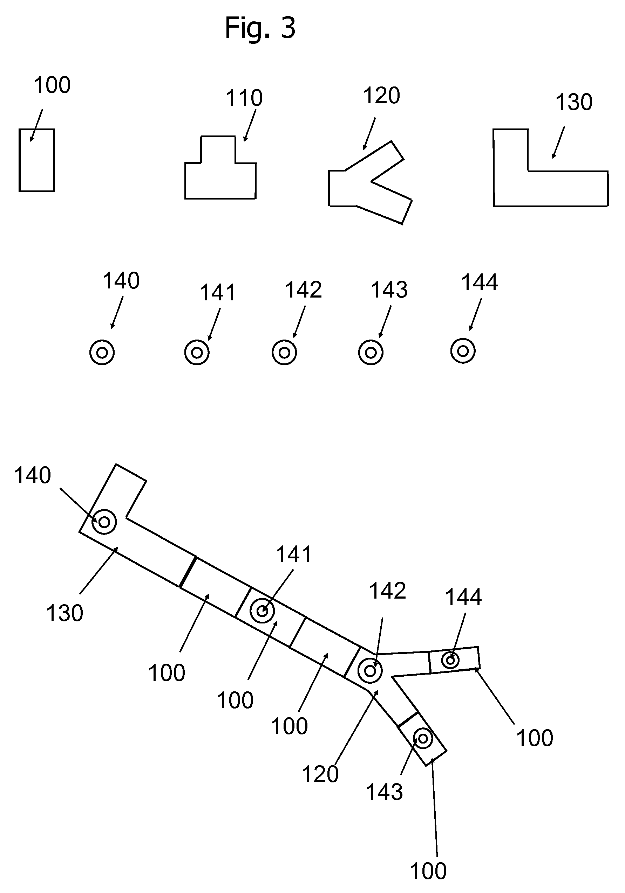

In einem vierten Schritt 4 wird mit Hilfe des mehrdimensionalen Modells des verletzten Körperteils ein mehrdimensionales Modell des gesunden Körperteils erzeugt. Insbesondere wird ein dreidimensionales Modell des gesunden Körperteils erstellt. Das mehrdimensionale Modell des gesunden Körperteils ermöglicht es, in einem fünften Schritt 5 ein Modell des Implantats zu entwerfen. Hierbei kann der Projektmitarbeiter Anpassungen am mehrdimensionalen Modell verletzten Körperteils vornehmen, die für die Behandlung des verletzten Körperteils von Bedeutung sind. Das mehrdimensionale Modell des Implantats kann in einem sechsten Schritt 6 an die Belastung des gesunden Körperteils angepasst werden, oder an Dimensionen, die typischerweise im vorgesehenen Defektbereich zur Anwendung kommen, sodass ein modifiziertes mehrdimensionales Modell des Implantats erhalten wird. Ein Beispiel für die Generierung eines modifizierten mehrdimensionalen Modells des Implantats ist in Fig. 3 gezeigt.

In einem siebenten Schritt 7 wird das modifizierte mehrdimensionale Modell des Implantats in eine Druckdatei für additives Herstellungsverfahren umgewandelt. Insbesondere kann in diesem Schritt eine Druckerdatei erzeugt werden, die eine Mehrzahl von Anweisungen zur Steuerung eines additiven Herstellungsverfahrens enthält.

In einem achten Schritt 8 werden aus der Druckdatei Anweisungen zur schichtweisen Herstellung des Implantats durch ein additives Herstellungsverfahren in einer additiven Herstellungsvorrichtung erstellt werden. Aus der Druckerdatei werden beispielsweise die Führung des Laserstrahls oder Elektronenstrahls sowie die Anzahl der im additiven Herstellungsverfahren zu druckenden Schichten ermittelt.

In einem neunten Schritt 9 wird das Implantat in der additiven Herstellungsvorrichtung hergestellt. Insbesondere wird das Implantat in der additiven Herstellungsvorrichtung schichtweise gemäss der in der Druckdatei für das additive Herstellungsverfahren bereitgestellten Anweisungen zur Herstellung des Implantats hergestellt. Die Herstellung des Implantats erfolgt insbesondere schichtweise im Sinterverfahren oder Schmelzverfahren.

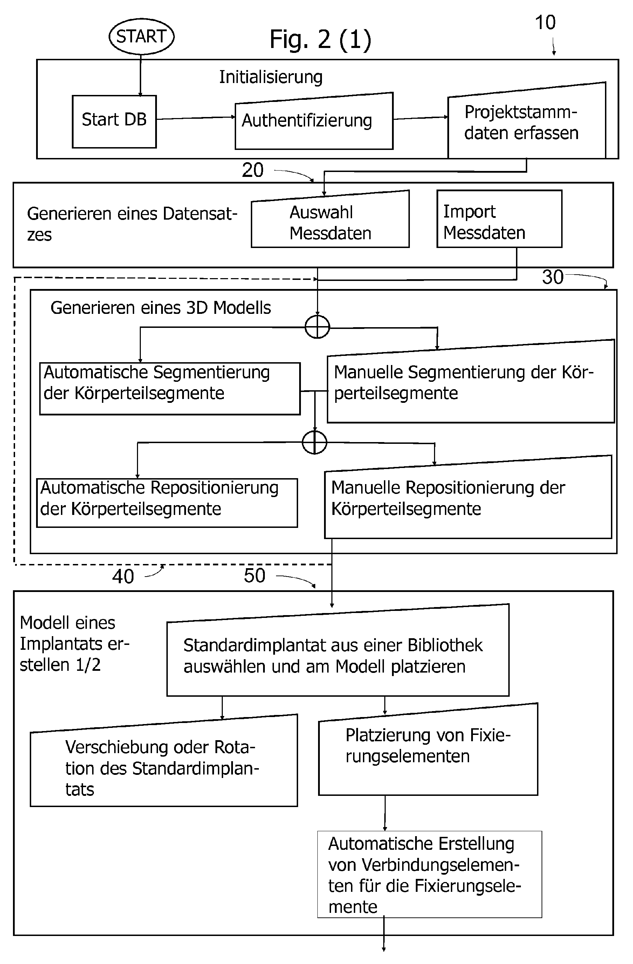

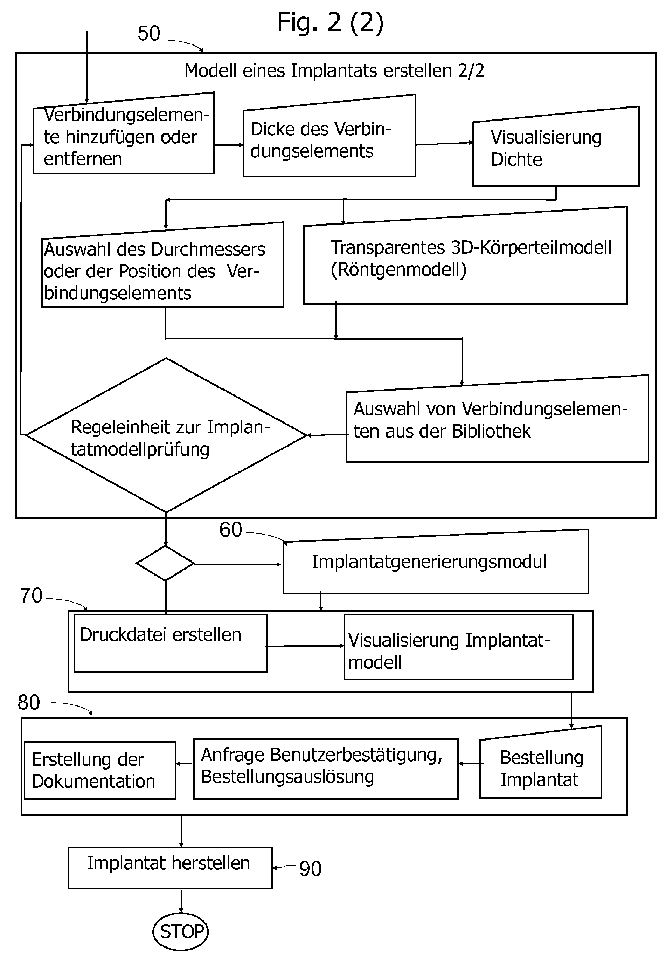

Das System zur Herstellung eines patientenspezifischen Implantats gemäss Fig. 2 umfasst die nachfolgenden Systemkomponenten, wobei die Beschreibung nicht ausschliessen soll, dass weitere Systemkomponenten oder Bausteine in das System mit aufgenommen werden können. Eine Systemkomponente kann ein Baustein oder ein Modul sein, welches Bestandteil des Systems ist. Fig. 2 erstreckt sich über zwei Seiten, wobei der erste Teil der Fig. 2 mit Fig. 2 (1) und der zweite Teil der Fig. 2 mit Fig. 2 (2) bezeichnet wird.

Eine Initialisierungseinheit 10 dient als eine erste Systemkomponente der Einleitung der Herstellung eines patientenspezifischen Implantats. Um die Herstellung eines Implantats zu initialisieren, wird in einem ersten Schritt ein Projekt in einer Projektdatenbank angelegt. Ein Projektmitarbeiter öffnet in einem ersten Schritt die Projektdatenbank, indem er sich bei der Projektdatenbank anmeldet. Die Anmeldung erfolgt durch eine Authentifizierung üblicherweise über die Eingabe eines Benutzernamens sowie der Eingabe eines Passworts. Es erfolgt eine Überprüfung, ob der Projektmitarbeiter bereits als Benutzer der Datenbank registriert ist, das heisst den Status eines registrierten Benutzers geniesst. Als registrierter Benutzer hat der Projektmitarbeiter auch Zugriff auf sämtliche durch ihn zu einem früheren Zeitpunkt angelegte Projektdaten. Der Projektmitarbeiter kann ein neues Projekt generieren, neue Patientendaten erzeugen oder bestehende Patienten einem neuen Projekt zuordnen. Das Projekt wird mit einer eindeutigen Projektnummer oder einer Projektkennung versehen. Ab diesem Zeitpunkt kann der Projektmitarbeiter dem Projekt Patientendaten zuordnen, so dass anhand dieser Projektstammdaten das Projekt vom System eindeutig identifiziert werden kann. Der Projektmitarbeiter kann auch bestehende Projektstammdaten modifizieren oder ergänzen.

Derartige Patientendaten können Bilddaten umfassen, wie durch ein bildgebendes Verfahren erzeugt worden sind. Insbesondere kann es sich bei den Patientendaten um Messdaten handeln, die am Patienten gemessen worden sind. Diese Messdaten können beispielsweise Computertomographie (CT) - Daten umfassen. Die Messdaten können mittels eines Datenträgers in die Datenbank transferiert werden, sodass mittels einer Messdatengenerierungseinheit 20 ein Messdatensatz generiert werden kann. Als Datenträger kann beispielsweise eine CD oder DVD oder ein USB Stick verwendet werden, die Daten können auch über ein Netzwerk beschafft oder transferiert werden.

Ein Modellgenerierungsmodul 30 erstellt ein mehrdimensionales Modell des verletzten Körperteils. Das Modellgenerierungsmodul 30 verwertet die Bilddaten des Messdatensatzes und erzeugt ein mehrdimensionales Modell des Körperteils oder einer Mehrzahl von Körperteilsegmenten des verletzten Körperteils. Der Körperteil ist zumeist bei Verwendung eines üblichen bildgebenden Messverfahrens aus der Mehrzahl an Körperteilsegmenten aufgebaut, so dass sich das mehrdimensionale Modell aus der Auswertung der Bilddaten der Körperteilsegmente des verletzten Körperteils ergibt. Der Projektmitarbeiter kann das mehrdimensionale Modell des verletzten Körperteils verändern, indem er den Körperteil oder einzelne Körperteilsegmente automatisch oder manuell positioniert oder ihre Position zueinander verändert.

Mittels eines Implantatauswahlmoduls 50 wählt der Projektmitarbeiter ein Modell aus einer Implantatdatenbank aus oder er erstellt ein mehrdimensionales Modell eines Implantats mittels eines Implantatgenerierungsmoduls 60. Jedes der Implantatauswahlmodule oder Implantatgenerierungsmodule kann die Manipulation des Durchtrennens oder Schneidens, des Verschiebens oder des Rotierens in der Perspektive des Betrachters des Bildschirms in der jeweiligen 2D-Bild-Ebene ermöglichen. Die 2D-Bild-Ebene wird durch den Bildschirm gebildet, sodass der Projektmitarbeiter durch Interaktion mit den am Bildschirm angezeigten Modellen die bestmögliche Lösung für ein patientenspezifisches Implantat ermitteln kann. Das Modellgenerierungsmodul 30 stellt ein mehrdimensionales Modell des verletzten Körperteils sowie dessen räumliche Anordnung auf dem Bildschirm als zweidimensionales Abbild der dreidimensionalen Anordnung des Körperteils oder der Körperteilsegmente dar. Das mehrdimensionale Modell zeigt insbesondere den Aufbau des verletzten Körperteils. Aufgrund der Visualisierung des mehrdimensionalen Modells auf dem Bildschirm kann der Projektmitarbeiter entscheiden, ob er eine Manipulation des mehrdimensionalen Modells vornehmen will.

Ein Modellvergleichsmodul 40 kann der Erstellung eines mehrdimensionalen Modells des gesunden Körperteils dienen. Dieses Modellvergleichsmodul kann gemäss Fig. 2 dieselben Arbeitsschritte wie das Modellgenerierungsmodul aufweisen. Daher ist das Modellgenerierungsmodul als optionale Verzweigung im Anschluss an Modellierung mit Hilfe des Modellgenerierungsmoduls 30 dargestellt. Die möglichen Arbeitsschritte für das Erstellen eines mehrdimensionalen Modells des gesunden Körperteils mittels des Modellvergleichsmoduls 40 entsprechen den Arbeitsschritten für das Erstellen eines mehrdimensionalen Modells des verletzten Körperteils mittels dem Modellgenerierungsmodul 30.

Das Implantatauswahlmodul 50 oder das Implantatgenerierungsmodul 60 dient der Erstellung eines mehrdimensionalen Modells eines Implantats, welches speziell an das mehrdimensionale Modell des verletzten oder gegebenenfalls des gesunden Körperteils angepasst ist. Das Implantatauswahlmodul 50 bedient sich einer Bibliothek von kommerziell erhältlichen Implantaten, aus denen der Projektmitarbeiter ein geeignetes Implantat auswählt. Das mehrdimensionale Modell des Implantats wird vom Projektmitarbeiter an der Stelle des mehrdimensionalen Modells des verletzten oder gegebenenfalls des gesunden Körperteils platziert, an welcher es für die Heilung des verletzten Körperteils benötigt wird. Der Projektmitarbeiter kann das Modell des Implantats durch Verschieben oder durch eine Rotationsbewegung an der Stelle des mehrdimensionalen Modells des Körperteils platzieren, welche durch die Verletzung geschädigt oder geschwächt ist. Insbesondere kann der Projektmitarbeiter an dieser Stelle die in Fig. 3 beschriebenen Schritte ausführen. Gleichzeitig oder im Anschluss an die Ausrichtung des Implantats am Modell des Körperteils erfolgt die Platzierung und Positionierung von Fixierungselementen. Die Fixierungselemente dienen der Befestigung des Implantats am Körperteil oder Körperteilsegment. Alternativ kann auch die Positionierung der Fixierungselemente vor der Erstellung der das Implantat ausbildenden Verbindungselemente vorgenommen werden. Die Verbindungselemente werden nach der Festlegung der Position und Dimension der Fixierungselemente automatisch als mehrdimensionales Modell generiert, sodass die Verbindungselemente ein mehrdimensionales Modell des Implantats ausbilden. Die automatische Generierung des mehrdimensionalen Modells der Verbindungselemente umfasst die Messung der Abstände und der Winkel zwischen den Fixierungselementen, die Erzeugung von Bohrungen zur Aufnahme der Fixierungselemente, die Berechnung der Länge, Breite und Wandstärke jedes Verbindungselements und gegebenenfalls eines Knotenelements in Abhängigkeit von den auf den Körperteil einwirkenden Belastungen. Die Belastungen, die auf den Körperteil einwirken, werden beispielsweise anhand der Dichteverteilung des Gewebes des Körperteils, beispielsweise der Knochendichteverteilung des mehrdimensionalen Modells des verletzten Knochens ermittelt. Insbesondere liefert die Knochendichteverteilung Hinweise auf die Spannungsverteilung im Knochen. Sie kann als Randbedingung in ein Finite-Elemente Modell des Implantats Eingang finden, sodass mittels des Finite-Elemente Modells des Implantats dessen optimale Konfiguration ermittelt werden kann. Das Ergebnis des Finite-Elemente Modells kann mittels des mehrdimensionalen Modells des Körperteils manuell durch den Projektmitarbeiter angepasst werden, wobei insbesondere zusätzliche Verbindungselemente vorgesehen werden können oder nicht benötigte Verbindungselemente entfernt werden können. Des Weiteren kann die Wandstärke des Implantats an die Einbausituation angepasst werden. Entsprechend der Abmessungen der Fixierungselemente kann auch der Durchmesser der Bohrung im Verbindungselement, welcher das Fixierungselement aufnimmt, angepasst werden. Insbesondere kann die Knochendichteverteilung auf der Oberfläche oder im Inneren des mehrdimensionalen Modells des Knochens farblich gekennzeichnet werden. Jeder Knochendichte wird bei deren Visualisierung in der Modellprojektion ein Farbton zugeordnet, sodass die Anordnung der Fixierungselemente manuell einfach überprüfbar ist.

Nicht nur die Anordnung der Fixierungselemente, auch deren Ausrichtung im Körperteil sowie deren Länge kann durch die Visualisierung am mehrdimensionalen Modell des Körperteils bestimmt werden. Die Ausrichtung und Länge der Fixierungselemente kann durch Berechnung automatisch ermittelt werden oder vom Projektmitarbeiter manuell eingestellt werden. Die Fixierungselemente sind ein Ausführungsbeispiel für Modulelemente gemäss Fig. 3. Insbesondere kann die Systemkomponente eine Visualisierung umfassen, bei welcher das dreidimensionale Modell als transparente Struktur dargestellt wird. In dieser Visualisierung ist es möglich, das Volumen und die Abmessungen des Körperteils darzustellen. Wahlweise kann das Körperteilvolumen eine Gewebedichteverteilung aufweisen, oder eine Knochendichteverteilung, sodass die Länge, der Durchmesser oder die Ausrichtung des Fixierungselements anhand der Visualisierung des Körperteilvolumens manuell angepasst werden kann. Ebenso kann anhand der Visualisierung des Körperteilvolumens etwa die räumliche Lage beispielsweise von Nerven, Zähnen, Sehnen oder anderen zur Definition des mehrdimensionalen Implantates relevanten Strukturen, beispielsweise Gewebestrukturen dargestellt werden. Für jede gewählte Modellvariante kann mittels eines Kontrollsystems, welches eine Regeleinheit enthält, zu jedem Zeitpunkt überprüft werden, ob die Modellvariante des Implantats, dessen Verbindungselemente oder dessen Fixierungselemente innerhalb der zulässigen Höchstbelastungen liegen. Sollte das Kontrollsystem eine Überschreitung eines Grenzwerts feststellen, wird eine Warnmeldung, ein visuelles oder akustisches Warnsignal erzeugt, um den Projektmitarbeiter darauf aufmerksam zu machen, dass durch das mehrdimensionale Modell des Implantats einer oder mehrere Grenzwerte nicht eingehalten werden.

Ein Druckdateierstellungsmodul 70 erhält das modifizierte mehrdimensionale Modell des Implantats vom Implantatauswahlmodul 50 oder vom Implantatgenerierungsmodul 60. Das Druckdateierstellungsmodul 70 wandelt das modifizierte mehrdimensionale Modell des Implantats in eine Druckdatei für ein additives Herstellungsverfahren um.

Mittels eines Anweisungsgenerierungsmoduls 80 werden aus der Druckdatei Anweisungen zur schichtweisen Herstellung des Implantats durch ein additives Herstellungsverfahren in einer additiven Herstellungsvorrichtung 90 erstellt. Die Herstellung des Implantats erfolgt in der additiven Herstellungsvorrichtung.

Fig. 3 zeigt die Schritte zur Erstellung eines modifizierten mehrdimensionalen Implantatmodells, welches mittels des Implantatauswahlmoduls 50 oder des Implantatgenerierungsmoduls 60 erstellt werden kann. Das Implantatauswahlmodul ermöglicht es dem Projektmitarbeiter, sein Implantat aus einer Bibliothek von Implantaten auszuwählen. Für die in der Bibliothek hinterlegten Implantate ist auch ein zugehöriges Belastungsprofil hinterlegt. Je nach verwendetem Werkstoff für das Implantat kann dieses einer unterschiedlichen Dauerbelastung standhalten und kann passend zu dem mehrdimensionalen Modell des Körperteils ausgewählt werden. Ein derartiges Implantat kann als Standardbaustein als ein Modulelement 100 ausgebildet sein. Fig. 3 zeigt schematisch eine stark vereinfachte Darstellung derartiger Modulelemente 100, 110, 120, 130 sowie eine Mehrzahl von Fixierungselementen 140, 141, 142, 143, 144. Ein modifiziertes mehrdimensionales Implantatmodell kann auch aus mehreren Modulelementen aufgebaut werden. In Fig. 3 ist ein modifiziertes mehrdimensionales Implantatmodell dargestellt, welches aus fünf Modulelementen 100 besteht, sowie aus einem Modulelement 120, welches ein Verzweigungselement umfasst und einem Modulelement 130, welches eine L-Form aufweist. Diese Modulelemente werden durch den Projektmitarbeiter aneinander angepasst. Gemäss der vorliegenden Darstellung haben die Modulelemente mindestens je eine Kantenlänge mit einem anderen Modulelement gemeinsam. Die Belastung an der gemeinsamen Kantenlänge von zwei benachbarten Modulelementen stimmt überein, ansonsten ist eine Auswahl des entsprechenden Modulelements nicht möglich. Das heisst, die Kantenlänge ist eine durch die Form des Modulelements bedingte Trennung, die im modifizierten mehrdimensionalen Implantatmodell nicht mehr sichtbar ist, da das aus dem modifizierten mehrdimensionalen Implantatmodell herzustellende Implantat als ein einziges, zusammenhängendes Bauteil hergestellt wird. Jeder der Kantenlängen ist ein Belastungszustand zugeordnet. Die Belastungszustände benachbarter Kantenlängen müssen übereinstimmen, ansonsten kann das modifizierte mehrdimensionale Implantatmodell nicht erstellt werden. Die Festigkeit des Modulelements wird so ausgewählt, dass sie mindestens der Festigkeit bei einer maximalen Belastung des Körperteils entspricht, sodass die Belastung, der das Implantat im Gebrauchszustand über seine gesamte Lebensdauer maximal ausgesetzt ist, kleiner als die Dauerfestigkeit des Modulelements ist.

Die Erfindung ist nicht auf die vorliegenden Ausführungsbeispiele beschränkt. Die Verfahrensschritte können insbesondere für beliebige Implantate für beliebige Körperteile oder Gliedmassen verwendet werden. Insbesondere können die Implantate aus den genannten Werkstoffen oder aus einer Kombination der genannten Werkstoffe hergestellt sein.

Für den Fachmann ist offensichtlich, dass viele weitere Modifikationen zusätzlich zu den beschriebenen Ausführungsbeispielen möglich sind, ohne vom erfinderischen Konzept abzuweichen. Der Gegenstand der Erfindung wird somit durch die vorangehende Beschreibung nicht eingeschränkt und ist durch den Schutzbereich bestimmt, der durch die Ansprüche festgelegt ist. Für die Interpretation der Ansprüche oder der Beschreibung ist die breitest mögliche Lesart der Ansprüche massgeblich. Insbesondere sollen die Begriffe "enthalten" oder "beinhalten" derart interpretiert werden, dass sie sich auf Elemente, Komponenten oder Schritte in einer nicht-ausschliesslichen Bedeutung beziehen, wodurch angedeutet werden soll, dass die Elemente, Komponenten oder Schritte vorhanden sein können oder genutzt werden können, dass sie mit anderen Elementen, Komponenten oder Schritten kombiniert werden können, die nicht explizit erwähnt sind. Wenn die Ansprüche sich auf ein Element oder eine Komponente aus einer Gruppe beziehen, die aus A, B, C... N Elementen oder Komponenten bestehen kann, soll diese Formulierung derart interpretiert werden, dass nur ein einziges Element dieser Gruppe erforderlich ist, und nicht eine Kombination von A und N, B und N oder irgendeiner anderen Kombination von zwei oder mehr Elementen oder Komponenten dieser Gruppe.

## Patentansprüche

1. Verfahren zur Herstellung eines patientenspezifischen Implantats, wobei eine Verletzung eines Körperteils eines Patienten mittels eines bildgebenden Verfahrens gemessen wird, ein Messdatensatz des verletzten Körperteils erzeugt wird, der einem mehrdimensionalen Abbild der Verletzung des Körperteils entspricht, die mit dem bildgebenden Verfahren ermittelt worden ist, aus dem Messdatensatz ein mehrdimensionales Modell des verletzten Körperteils erzeugt wird, wobei mit Hilfe des mehrdimensionalen Modells des verletzten Körperteils ein Modell des gesunden Körperteils erzeugt werden kann, welches insbesondere die Kräfte enthält, welche durch eine Dauerbelastung auf das Körperteil wirken,
wobei ein mehrdimensionales Modell des Implantats erstellt wird,
**dadurch gekennzeichnet, dass** das mehrdimensionale Modell des Implantats an das mehrdimensionale Modell des Körperteils angepasst wird, sodass ein modifiziertes mehrdimensionales Modell des Implantats erhalten wird,
wobei das modifizierte mehrdimensionale Modell des Implantats aus mindestens einem Modulelement generiert wird,
wobei die Festigkeit des Modulelements so ausgewählt wird, dass sie mindestens der Festigkeit bei einer maximalen Belastung des Körperteils entspricht, sodass die Belastung, der das Implantat im Gebrauchszustand über seine gesamte Lebensdauer maximal ausgesetzt ist, kleiner als die Dauerfestigkeit des Modulelements ist.

2. Verfahren nach Anspruch 1, wobei das modifizierte mehrdimensionale Implantatmodell mittels eines Implantatauswahlmoduls (50) oder eines Implantatgenerierungsmoduls (60) erzeugt wird, wobei mittels des Implantatauswahlmoduls (50) ein Implantat aus einer Bibliothek von Implantatmodellen ausgewählt werden kann, wobei das ausgewählte Implantatmodell mittels des Modellgenerierungsmoduls oder des Modellvergleichsmoduls an das mehrdimensionale Modell des verletzten oder gesunden Körperteils angepasst wird und/oder mittels des Implantatauswahlmoduls oder des Implantatgenerierungsmoduls, mittels welchem das mehrdimensionale Implantatmodell derart erzeugt werden kann, dass das für die vorliegende Verletzung speziell entwickelte modifizierte mehrdimensionale Implantatmodell aus dem Modulelement generiert werden kann, sodass mit zumindest einem der Implantatauswahlmodule oder Implantatgenerierungsmodule das modifizierte mehrdimensionale Modell des Implantats erzeugt werden kann.

3. Verfahren nach einem der vorhergehenden Ansprüche, wobei das modifizierte mehrdimensionale Modell des Implantats eine Mehrzahl von Modulelementen enthält, wobei die Modulelemente an das modifizierte mehrdimensionale Modell des verletzten Körperteils, des gesunden Körperteils oder des Implantats in der Perspektive des Betrachters in einer zweidimensionalen Bildebene angepasst werden können, wobei mittels der Modulelemente zumindest eine der Operationen eines Durchtrennens, Schneidens, eines Verschiebens, Zusammenfügens, Skalierens, Spiegelns, Zusammenschiebens, Aufklappens, Entfaltens oder einer Rotation erfolgen kann.

4. Verfahren zur Herstellung des patientenspezifischen Implantats nach einem der vorhergehenden Ansprüche, wobei
(1) in einem ersten Schritt die Verletzung eines Körperteils eines Patienten mittels des bildgebenden Verfahrens gemessen wird,
(2) in einem zweiten Schritt der Messdatensatz erzeugt wird, der einem mehrdimensionalen Abbild der Verletzung des Körperteils entspricht, die mit dem bildgebenden Verfahren ermittelt worden ist,
(3) in einem dritten Schritt aus dem Messdatensatz das mehrdimensionales Modell des verletzten Körperteils erzeugt wird,
(4) in einem vierten Schritt mit Hilfe des mehrdimensionalen Modells des verletzten Körperteils das Modell des gesunden Körperteils erzeugt wird,
(5) in einem fünften Schritt das mehrdimensionales Modell eines Implantats erstellt wird,
(6) in einem sechsten Schritt das mehrdimensionale Modell des Implantats an das mehrdimensionale Modell des verletzten oder gesunden Körperteils angepasst wird, sodass das modifizierte mehrdimensionale Modell des Implantats erhalten wird,
(7) in einem siebenten Schritt das modifizierte mehrdimensionale Modell des Implantats in eine Druckdatei für ein additives Herstellungsverfahren umgewandelt wird oder in ein Anweisungsmodul für eine Herstellungsanweisung für ein subtraktives Herstellungsverfahren umgewandelt wird,
(8) gegebenenfalls in einem achten Schritt aus der Druckdatei Anweisungen zur schichtweisen Herstellung des Implantats durch ein additives Herstellungsverfahren in einer additiven Herstellungsvorrichtung (90) erstellt werden oder aus der Herstellungsanweisung Anweisungen zur Herstellung des Implantats in einem subtraktiven Herstellungsverfahren erstellt werden,
(9) in einem neunten Schritt das Implantat in der additiven Herstellungsvorrichtung hergestellt wird oder das Implantat gemäss Herstellungsanweisung in einem subtraktiven Herstellungsverfahren hergestellt wird, wobei
im fünften Schritt (5) mit einem Implantatauswahlmodul (50) ein mehrdimensionales Modell eines Implantats ausgewählt wird oder im sechsten Schritt (6) mit einem Implantatgenerierungsmodul (60) ein mehrdimensionales Modell eines Implantats erzeugt wird, wobei das mehrdimensionale Modell des Implantats an das mehrdimensionale Modell des verletzten oder gesunden Körperteils angepasst wird, sodass das modifizierte mehrdimensionale Modell des Implantats erhalten wird.

5. Verfahren nach Anspruch 4, wobei zumindest eines der mehrdimensionalen Modelle des verletzten Körperteils, des gesunden Körperteils oder des Implantats in der Perspektive des Betrachters in einer zweidimensionalen Bildebene manipuliert wird, wobei die Manipulation zumindest eine der Operationen eines Durchtrennens, Schneidens, Zusammenfügens, Skalierens, Spiegelns, Aufklappens, Entfaltens, Verschiebens, Zusammenschiebens, oder eines Rotierens zumindest eines der mehrdimensionalen Modelle des verletzten Körperteils, des gesunden Körperteils oder des Implantats umfassen kann.

6. Verfahren nach Anspruch 5, wobei im zweiten Schritt (2) das Messergebnis nach Erzeugen eines Projekts in einer Initialisierungseinheit (10) in eine Messdatengenerierungseinheit (20) eingespeist wird, wobei ein Messdatensatz erzeugt wird, der einem mehrdimensionalen Abbild der Verletzung des Körperteils entspricht, die mit dem bildgebenden Verfahren ermittelt worden ist und/oder wobei im dritten Schritt (3) aus dem Messdatensatz ein mehrdimensionales Modell des verletzten Körperteils in einem Modellgenerierungsmodul (30) erzeugt werden kann und/oder wobei im vierten Schritt mit Hilfe des mehrdimensionalen Modells des verletzten Körperteils in einem Modellvergleichsmodul (40) ein Modell des gesunden Körperteils erzeugt werden kann.

7. Verfahren nach einem der vorhergehenden Ansprüche, wobei beim Laden der Messdatendatei durch die Modellierungssoftware ein mehrdimensionales Modell des oder der verletzten Körperteile erstellt wird, wobei dieses mehrdimensionale Modell als ein Oberflächenmodell ausgebildet ist, mittels welchem der verletzte Körperteil in der Lage angezeigt wird, welche der verletzte Körperteil bei Durchführung des bildgebenden Verfahrens hatte, sodass die Lage des mehrdimensionalen Modells des Körperteils am Bildschirm derart verändert werden kann, dass die gewünschte Ansicht am Bildschirm angezeigt wird, wobei die Lage von Teilen des mehrdimensionalen Modells des Körperteils am Bildschirm derart verändert wird, dass sie in der gewünschten Lage zueinander liegen.

8. System zur Herstellung eines Implantats umfassend eine Initialisierungseinheit (10) zur Erzeugung eines Projekts, eine Messdatengenerierungseinheit (20), mittels welcher ein Messdatensatz aus einer Messung am Patienten mittels eines bildgebenden Verfahrens generierbar ist, wobei der Messdatensatz ein Abbild eines verletzten Körperteils enthält, welches mittels eines bildgebenden Messverfahrens erstellt ist, ein Modellgenerierungsmodul (30), mittels welchem ein mehrdimensionales Modell des verletzten Körperteils aus dem Messdatensatz generierbar ist, gegebenenfalls ein Modellvergleichsmodul (40), mittels welchem ein mehrdimensionales Modell eines gesunden Körperteils generierbar ist, ein Implantatauswahlmodul (50), wobei mittels des Implantatauswahlmoduls (50) ein Implantat aus einer Bibliothek von Implantatmodellen derart auswählbar ist, dass das Implantatmodell mittels des Modellgenerierungsmoduls oder des Modellvergleichsmoduls an das mehrdimensionale Modell des verletzten oder gesunden Körperteils anpassbar ist und/oder mittels einem Implantatgenerierungsmodul (60), mittels welchem ein mehrdimensionales Modell eines Implantats derart erzeugbar ist, dass ein für die spezifische Verletzung neu entwickeltes mehrdimensionales Implantatmodell aus mindestens einem Modulelement generierbar ist, sodass mit zumindest einem der Implantatauswahlmodule oder Implantatgenerierungsmodule (50, 60) ein modifiziertes mehrdimensionales Modell des Implantats erhältlich ist,
**dadurch gekennzeichnet, dass**
das mehrdimensionale Modell des Implantats an das mehrdimensionale Modell des Körperteils angepasst wird, sodass das modifizierte mehrdimensionale Modell des Implantats erhältlich ist,
wobei das modifizierte mehrdimensionale Modell des Implantats aus dem mindestens einen Modulelement erzeugbar ist,
wobei die Festigkeit des Modulelements auswählbar ist, dass sie mindestens der Festigkeit bei einer maximalen Belastung des Körperteils entspricht, sodass die Belastung, der das Implantat im Gebrauchszustand über seine gesamte Lebensdauer maximal ausgesetzt ist, kleiner als die Dauerfestigkeit des Modulelements ist.

9. System nach Anspruch 8, wobei die Modulelemente als ein Baukastensystem ausgebildet sind, wobei mindestens je zwei Modulelemente auf einer gemeinsamen Passungsfläche derart zusammenfügbar sind, dass die über die Passungsfläche übertragene Belastung übereinstimmt.

10. System nach einem der Ansprüche 8 oder 9, wobei das aus den Modulelementen erzeugte modifizierte mehrdimensionale Modell des Implantats in ein Druckdateierstellungsmodul (70) für ein additives Herstellungsverfahren aufnehmbar ist oder in ein Anweisungsmodul für eine Herstellungsanweisung für ein subtraktives Herstellungsverfahren umwandelbar ist.

11. System nach einem der Ansprüche 8 bis 10, wobei das Implantatgenerierungsmodul zumindest ein Element aus der Gruppe der Trennmodule, der Schneidmodule, der Verschiebemodule, der Zusammenfügemodule, der Skaliermodule, der Spiegelmodule, der Module zum Zusammenschieben, der Module zum Aufklappen, der Module zum Entfalten oder der Rotationsmodule enthält.

12. System nach einem der Ansprüche 8 bis 11, wobei die Dauerfestigkeit des mittels des Implantatauswahlmoduls (50) ausgewählten modifizierten mehrdimensionalen Modells des Implantats oder des mittels des Implantatgenerierungsmoduls (60) entwickelten modifizierten mehrdimensionalen Modells des Implantats aus der Dauerfestigkeit der Modulelemente ermittelbar ist und mittels eines Belastungsintensitätsmoduls überprüfbar ist, ob die Dauerfestigkeit des modifizierten mehrdimensionalen Modells des Implantats für die Dauerbelastung des Implantats im Körperteil ausreichend ist.

13. System zur Herstellung eines Implantats nach einem der vorhergehenden Ansprüche 8 bis 12, umfassend die Initialisierungseinheit (10) zur Erzeugung eines Projekts, die Messdatengenerierungseinheit (20), mittels welcher der Messdatensatz aus einer Messung am Patienten mittels des bildgebenden Verfahrens generierbar ist, wobei der Messdatensatz ein Abbild des verletzten Körperteils enthält, welches mittels eines bildgebenden Messverfahrens erstellt ist, ein Modellgenerierungsmodul (30), mittels welchem ein mehrdimensionales Modell des verletzten Knoches aus dem Messdatensatz generierbar ist, gegebenenfalls ein Modellvergleichsmodul (40), mittels welchem ein mehrdimensionales Modell eines gesunden Körperteils generierbar ist, ein Implantatauswahlmodul (50), wobei mittels des Implantatauswahlmoduls (50) ein Implantat aus einer Bibliothek von Implantatmodellen derart auswählbar ist, dass das Implantatmodell mittels des Modellgenerierungsmoduls oder des Modellvergleichsmoduls an das mehrdimensionale Modell des verletzten oder gesunden Körperteils anpassbar ist und/oder mittels einem Implantatgenerierungsmodul (60), mittels welchem ein mehrdimensionales Modell eines Implantats derart erzeugbar ist, sodass ein für die spezifische Verletzung neu entwickeltes mehrdimensionales Implantatmodell generierbar ist, sodass mit zumindest einem der Implantatauswahlmodule oder Implantatgenerierungsmodule (50, 60) ein modifiziertes mehrdimensionales Modell des Implantats erhältlich ist, wobei das Implantatmodell in ein Druckdateierstellungsmodul (70) für ein additives Herstellungsverfahren aufnehmbar ist, wobei ein Anweisungsgenerierungsmodul (80) vorgesehen ist, mittels welchem eine vom Druckdateierstellungsmodul (70) erstellte Druckdatei in eine Herstellungsanweisung für eine additive Herstellungsvorrichtung (90) umwandelbar ist, wobei die additive Herstellungsvorrichtung zur Herstellung des Implantats gemäss der Herstellungsanweisung vorgesehen ist, **dadurch gekennzeichnet, dass** durch zumindest eines der Implantatauswahlmodule (50) oder Implantatgenerierungsmodule (60) ein patientenspezifisches Implantat aus dem modifizierten mehrdimensionalen Modell des Implantats erhältlich ist, wobei mit dem Implantatauswahlmodul (50) das mehrdimensionale Modell des Implantats ausgewählt werden kann oder mit dem Implantatgenerierungsmodul (60) das mehrdimensionale Modell des Implantats erzeugt werden kann, wobei das mehrdimensionale Modell des Implantats an das mehrdimensionale Modell des verletzten oder gesunden Körperteils anpassbar ist, sodass das modifizierte mehrdimensionale Modell des Implantats erhältlich ist.

14. System nach Anspruch 13, wobei das modifizierte mehrdimensionale Modell des Implantats mittels eines Druckdateierstellungsmoduls (70) in eine Druckdatei für ein additives Herstellungsverfahren umwandelbar ist und/oder mittels eines Anweisungsgenerierungsmoduls (80) aus der Druckdatei Anweisungen zur schichtweisen Herstellung des Implantats durch ein additives Herstellungsverfahren in der additiven Herstellungsvorrichtung (90) erstellbar ist und/oder wobei mittels dem Implantatauswahlmodul oder dem Implantatgenerierungsmodul (50, 60) zumindest eines der mehrdimensionalen Modelle des verletzten Körperteils, des gesunden Körperteils oder des Implantats in der Perspektive des Betrachters in einer zweidimensionalen Bildebene mittels eines Implantatbearbeitungsmoduls manipulierbar sein kann.

15. System nach einem der Ansprüche 13 oder 14, wobei das mittels des Implantatauswahlmoduls (50) ausgewählte Implantatmodell oder das mit dem Implantatgenerierungsmodul (60) entwickelte Implantatmodell mittels eines Belastungsintensitätsmoduls auf das Vorhandensein von übermässigen Belastungen für den Körperteil überprüfbar ist.
